# EUROPEAN PATENT APPLICATION

(11) **EP 4 603 099 A1**
(43) Date of publication of application: **20.08.2025**
(21) Application number: 23874919.6
(22) Date of filing: 04.10.2023
(51) Int. Cl.: A61K 31/7064, A01N 43/90, A01P 1/00, A23K 20/163, A23L 33/10, A61K 8/60, A61K 8/69, A61P 31/16

(54) **ANTI-INFLUENZA VIRUS COMPOSITION, MEDICINE, FOOD, BEVERAGE, SUPPLEMENT, AGRICULTURAL CHEMICAL, FEED AND COSMETIC**

(30) Priority: 04.10.2022 US 202263412884 P
(71) Applicant: Igarashi, Manabu, Sapporo-shi, Hokkaido 060-0808 (JP); Kato, Hiroki, 53127 Bonn (DE); Tsukamoto, Yuta, 53127 Bonn (DE)
(72) Inventor: Igarashi, Manabu, Sapporo-shi, Hokkaido 060-0808 (JP); Kato, Hiroki, 53127 Bonn (DE); Tsukamoto, Yuta, 53127 Bonn (DE)
(74) Representative: Michalski Hüttermann & Partner Patentanwälte mbB
(86) International application number: PCT/JP2023/036277
(87) International publication number: WO 2024/075794

(57) **Abstract**

There is provided a novel anti-influenza virus agent that inhibits a mechanism of proliferation or infection of an influenza virus and thus has a target which is not a specific viral protein, where the novel anti-influenza virus agent does not cause the emergence of drug-resistant viruses. There are also provided a medicine, a food, a beverage, a supplement, an agricultural chemical, a feed, and a cosmetic, which use the anti-influenza virus agent. Provided is an anti-influenza virus composition containing, as an active ingredient, tubercidin, a tubercidin derivative, or a salt thereof. Also provided are a medicine, a food, a beverage, a supplement, an agricultural chemical, a feed, and a cosmetic, which use the anti-influenza virus agent.

## Description

### TECHNICAL FIELD

The present invention relates to a composition that can be used for an anti-influenza virus drug or the like, and a medicine, a supplement, an agricultural chemical, a feed, and a cosmetic, which use the composition.

Priority is claimed on United States Patent No. 63/412,884, filed October 4, 2022, the content of which is incorporated herein by reference.

### BACKGROUND ART

An epidemic of the influenza virus is regularly repeated among humans almost every year. In particular, the A type influenza virus is known as a zoonotic pathogen that widely infects mammals including humans and birds and often causes severe damage due to a global pandemic in society. There are also a number of reports that a virus prevalent in poultry is directly transmitted to humans, resulting in death.

Several drugs have been approved as anti-influenza virus agents for the treatment or the like of influenza. All of these directly act on a specific viral protein. For example, as a representative anti-influenza virus agent, a medicinal composition which contains, as an active ingredient, oseltamivir phosphate having an inhibitory activity against neuraminidase possessed by an influenza virus, has been developed.

For example, Patent Document 1 discloses a medicinal composition that contains one or more excipients selected from a sugar and a sugar alcohol, which have an equilibrium moisture content of 1% by weight or less at 25°C and a relative humidity of 70%, and contains oseltamivir phosphate, where the contents of glucose and mannose which are contained as impurities in the sugar and the sugar alcohol are each 0.01% by weight or less with respect to the sugar or the sugar alcohol. This technique aims to provide a medicinal composition containing oseltamivir phosphate having improved storage stability, particularly storage stability against humidity, temperature, and the like in a storage environment, particularly suppression of coloration during storage.

### Citation List

### Patent Document

Patent Document 1: PCT International Publication No. WO2007/097325

### SUMMARY OF INVENTION

### Technical Problem

However, a drug having an active ingredient that directly acts on a specific viral protein may have a possibility of the emergence of a drug-resistant virus due to mutation of a viral gene. A virus strain that is resistant to an anti-influenza virus agent targeting neuraminidase has also been found.

Therefore, there is a strong demand for the development of a novel anti-influenza virus agent that has a target other than the specific viral protein or has a mechanism other than directly acting on the specific viral protein, can suppress virus proliferation, and does not cause the emergence of drug-resistant viruses.

The present invention has been made in consideration of such a background, and an object of the present invention is to provide a novel anti-influenza virus agent that inhibits a mechanism of proliferation or infection of an influenza virus and thus has a target which is not a specific viral protein, where the novel anti-influenza virus agent does not cause the emergence of drug-resistant viruses. There are also provided a medicine, a food, a beverage, a supplement, an agricultural chemical, a feed, and a cosmetic, which use the anti-influenza virus agent.

### Solution to Problem

The present invention includes the following aspects.
[1] An anti-influenza virus composition containing, as an active ingredient, tubercidin, a tubercidin derivative, or a salt thereof.
[2] The composition according to [1],
   in which the tubercidin derivative is a compound represented by General Formula (1), 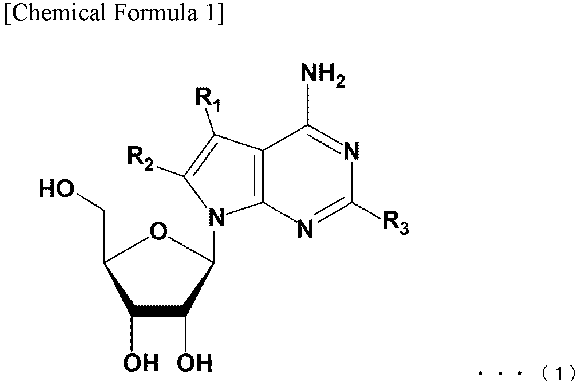
   (in General Formula (1), R₁, R₂, and R₃ are each the same or different from each other and represent a hydrogen atom, a halogen atom, an alkyl group which may have a substituent, an alkenyl group which may have a substituent, an alkynyl group which may have a substituent, an alkoxy group which may have a substituent, an amino group which may have a substituent, an amide group which may have a substituent, a cyano group, a nitro group, a hydroxy group, a sulfone group, a cycloalkyl group which may have a substituent, an aryl group which may have a substituent, an aliphatic heterocyclic group which may have a substituent, or an aromatic heterocyclic group which may have a substituent).
[3] The composition according to [2], in which R₁ and R₂ are each the same or different from each other and represent a hydrogen atom, a halogen atom, an amino group which may have a substituent, an amide group which may have a substituent, or a cyano group, and R₃ represents a hydrogen atom, an alkyl group which may have a substituent, an amino group which may have a substituent, or an aryl group which may have a substituent.
[4] The composition according to [2] or [3], in which R₃ represents a hydrogen atom.
[5] The composition according to any one of [2] to [4], in which R₁ and R₂ are each the same or different from each other and represent a halogen atom.
[6] The composition according to any one of [2] to [4], in which R₁ and R₂ are each the same or different from each other and represent an amino group which may have a substituent, or a cyano group.
[7] The composition according to any one of [2] to [4], in which R₁ represents an alkyl group substituted with a halogen.
[8] The composition according to any one of [2] to [4], in which R₁ represents a trifluoromethyl group.
[9] The composition according to [2], in which the tubercidin derivative is a compound represented by Formula (2), 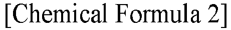 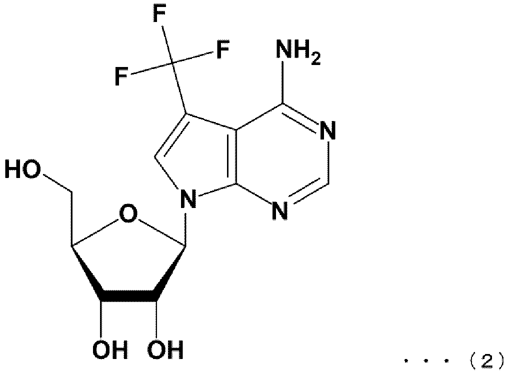
[10] A medicine, a food, a beverage, a supplement, an agricultural chemical, a feed, or a cosmetic, including:
   the composition according to any one of [1] to [9].

### Advantageous Effects of Invention

According to the present invention, it is possible to obtain a novel anti-influenza virus agent that inhibits a mechanism of proliferation or infection of an influenza virus and thus has a target which is not a specific viral protein, where the novel anti-influenza virus agent does not cause the emergence of drug-resistant viruses. It is possible to obtain a medicine, a food, a beverage, a supplement, an agricultural chemical, a feed, and a cosmetic, which use the anti-influenza virus agent.

### BRIEF DESCRIPTION OF DRAWINGS

[FIG. 1] FIG. 1 shows a schematic view showing an outline of a search for an MTr1 inhibitory compound.
[FIG. 2] FIG. 2 shows a photographic view of Western blot showing the generation of MTr1 knockout (KO) A549 cells by CRISPR/Cas9.
[FIG. 3] FIG. 3 shows graphs showing accumulation of IAV and IBV viruses in wild type and MTr1 KO A549 cells.

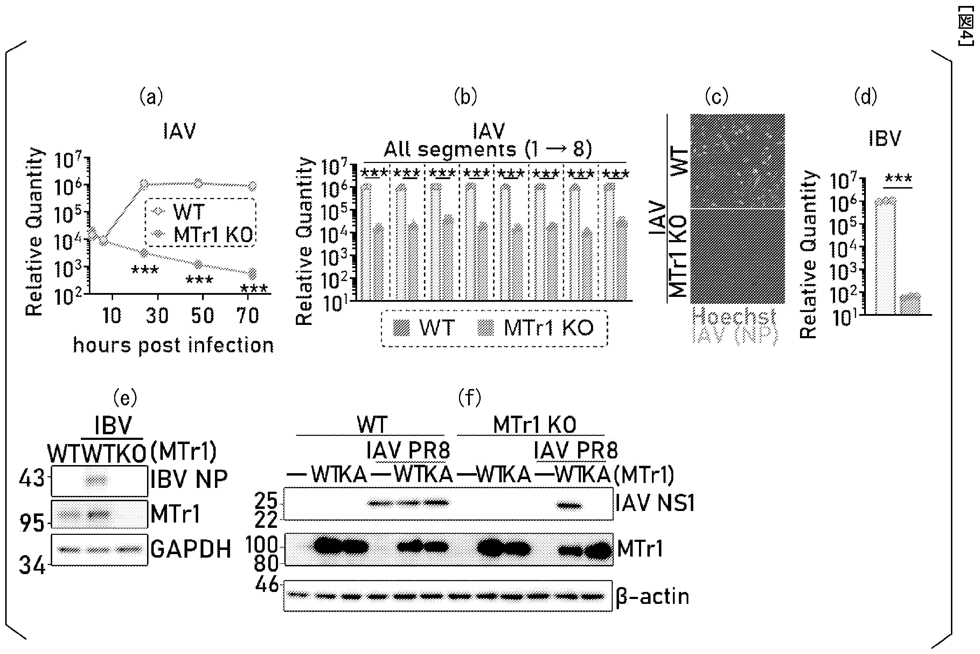

[FIG. 4] FIG. 4(a) shows a graph showing qRT-PCR analysis of IAV RNA in WT and MTr1 KO A549 cells, which have been infected with IAV PR8. FIG. 4(b) shows a graph showing qRT-PCR analysis of individual IAV RNA segments in WT and MTr1 KO A549 cells, which have been infected with IAV PR8 for 24 hours. FIG. 4(c) shows a photographic view showing immunostaining of a viral protein in WT and MTr1 KO A549 cells, which have been infected with IAV PR8 for 19 hours. FIG. 4(d) shows a graph showing qRT-PCR analysis of IBV RNA in WT and MTr1 KO A549 cells, which have been infected with IBV for 24 hours. FIG. 4(e) shows a photographic view showing Western blot analysis of the indicated proteins in WT A549 cells or MTr1 KO A549 cells infected with IBV for 24 hours. FIG. 4(f) shows a photographic view showing Western blot analysis of each protein in WT and MTr1 KO A549 cells, which have been infected with the IAV PR8 strain for 24 hours.
[FIG. 5] FIG. 5(a) shows a photographic view showing each immunostaining in WT and MTr1 KO A549 cells, which have been infected with the IAV PR8 strain for 24 hours. FIG. 5(b) shows graphs showing the results of flow cytometry in WT and MTr1 KO A549 cells, which have been infected with the IAV PR8 strain for 24 hours.
[FIG. 6] FIG. 6 shows graphs showing the chain-specific qRT-PCR analysis of IAV vRNA, mRNA, and cRNA in WT and MTr1 KO A549 cells, which have been infected with IAV PR8 for 24 hours.
[FIG. 7] FIG. 7 shows graphs showing the chain-specific qRT-PCR analysis of IAV vRNA, mRNA, and cRNA in WT and MTr1 KO A549 cells infected with IAV PR8 at each m.o.i. and each time.
[FIG. 8] FIG. 8 shows a graph showing qRT-PCR analysis of NT/U2-IAV hybrid RNA in WT and MTr1 KO A549 cells, which have been infected with IAV PR8 for 24 hours at each m.o.i.
[FIG. 9] FIG. 9(a) shows a schematic view showing primer sequences for cap snatching-specific qPCR. FIG. 9(b) shows graphs showing qRT-PCR analysis of IAV RNA, U2, and U2-IAV RNA hybrid RNA in A549 cells infected with IAV PR8 for 24 hours. FIG. 9(c) shows a graph showing the qRT-PCR analysis of the U2 RNA of the same sample as in FIG. 8.
[FIG. 10] FIG. 10 shows graphs showing qRT-PCR analysis and virus quantity of WT and MTr1 KO cells, which have been infected with IAV.
[FIG. 11] FIG. 11 shows a photographic view showing the results of Western blotting of WT and MTr1 KO cells, which have been infected with IAV. FIG. 11(a) shows the results in WT and MTr1 KO A549 cells, which have been infected with IAV WSN, and FIG. 11(b) shows the results in WT and MTr1 KO A549 cells, which have been infected with SC35M-GFP.
[FIG. 12] FIG. 12 shows graphs showing qRT-PCR analysis and virus quantity of WT and MTr1 KO cells, which have been infected with IDV or THOV. FIG. 12(a) shows the virus quantity in the supernatant of WT and MTr1 KO A549 cells, which have been infected with IDV. FIG. 12(b) shows the results of qRT-PCR analysis of THOV RNA in WT and MTr1 KO A549 cells, which have been infected with THOV.
[FIG. 13] FIG. 13 shows graphs showing virus quantity and RNA amount in WT and MTr1 KO A549 cells, which have been infected with various viruses. FIG. 13(a) shows the virus quantity in the supernatant from WT and MTr1 KO A549 cells, which have been infected with RVFV. FIG. 13(b) shows the results of qRT-PCR analysis of virus RNA in WT and MTr1 KO A549 cells, which have been infected with HAZV, ISKV, STBV, or DUGV.
[FIG. 14] FIG. 14(a) shows a schematic view showing a phylogenetic tree of an S segment of the bunya virus. FIG. 14(b) shows a photographic view showing the results of Western blot analysis of the indicated proteins in WT A549 cells or MTr1 KO A549 cells infected with HAZV. FIG. 14(c) shows graphs showing the results of qRT-PCR analysis of the designated RNA virus in A549 cells.
[FIG. 15] FIG. 15 shows a schematic view showing an outline of the dependence of Mtr1 on the replication of the cap snatching virus.
[FIG. 16] FIG. 16 shows a schematic view showing a schematic flowchart of primary screening.
[FIG. 17] FIG. 17(a) shows a schematic view showing a 2D interaction between SAM and human MTr1 (PDB ID: 4N49). FIG. 17(b) shows a schematic view of a screening strategy for acquiring an Mtr1 inhibitor candidate. FIG. 17(c) shows a schematic view showing docking scores and virtual screening rankings of representative compounds.
[FIG. 18] FIG. 18 shows a graph showing the results of qRT-PCR analysis of IAV RNA in A549 cells infected with the IAV PR8 strain for 24 hours.

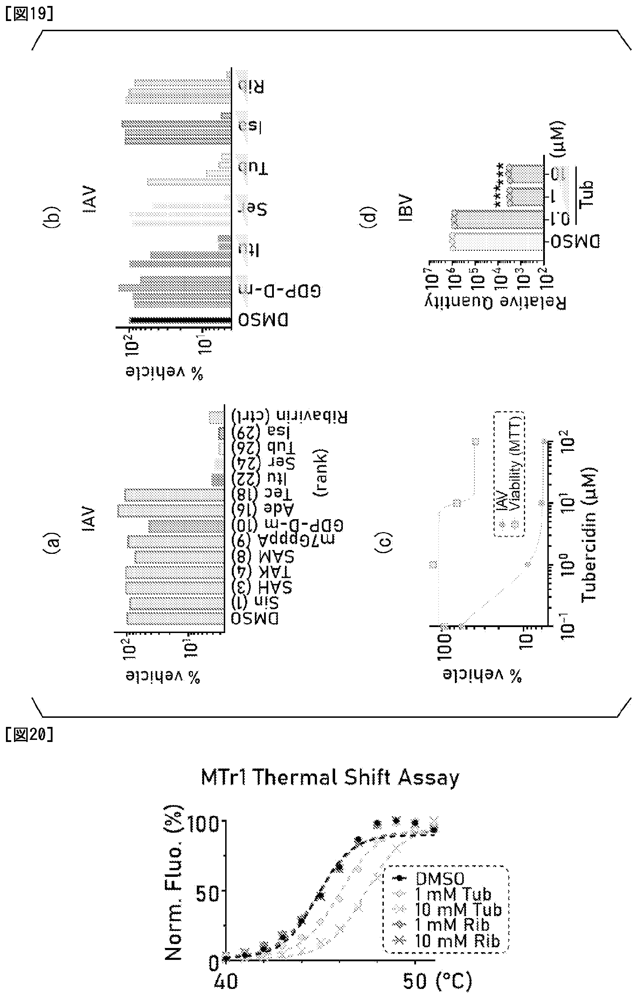

[FIG. 19] FIG. 19(a) shows a graph showing the results of quantification of the level of GFP in A549 cells infected with the IAV-GFP SC35M strain for 19 hours. FIG. 19(b) shows a graph showing the results of another quantification of the level of GFP in A549 cells infected with the IAV-GFP SC35M strain for 19 hours. FIG. 19(c) shows a graph showing the results of the quantification of the GFP level and the cell cytotoxicity test (WST-8) in A549 cells infected with IAV-GFP for 19 hours. FIG. 19(d) shows a graph showing the results of qRT-PCR analysis of IAV RNA in A549 cells infected with the IAV PR8 strain for 24 hours.
[FIG. 20] FIG. 20 shows a graph showing the results of a thermal shift assay of recombinant MTr1 using SYPRO Orange.
[FIG. 21] FIG. 21(a) shows a graph showing other results of the thermal shift assay of the recombinant MTr1 using SYPRO Orange. FIG. 21(b) shows a graph showing the results of quantification of an MTase assay using human MTr1 WT or MTr1 K239A, which contains SAM, SAH, or sinefungin. FIG. 21(c) shows a graph showing the results of the MTase assay using human MTr1 containing tubercidin at the indicated concentration.
[FIG. 22] FIG. 22(a) shows a schematic flowchart of a secondary screening process consisting of three steps. FIG. 22(b) shows a schematic view showing a docking state of TFMT in a SAM binding pocket of human MTr1. FIG. 22(c) shows a graph showing the results of a thermal shift assay of Mtr1 with 1 mM TFMT or ribavirin, which uses SYPRO Orange.
[FIG. 23] FIG. 23(a) shows a graph showing the results of an MTase assay of MTr1 with TFMT or ribavirin. FIG. 23(b) shows a graph showing the results of the qRT-PCR analysis of each virus RNA. FIG. 23(c) shows a graph showing the results of quantification of the GFP level in A549 cells infected with IAV-GFP. FIG. 23(d) shows a graph showing the results of another quantification of the GFP level in A549 cells infected with IAV-GFP.

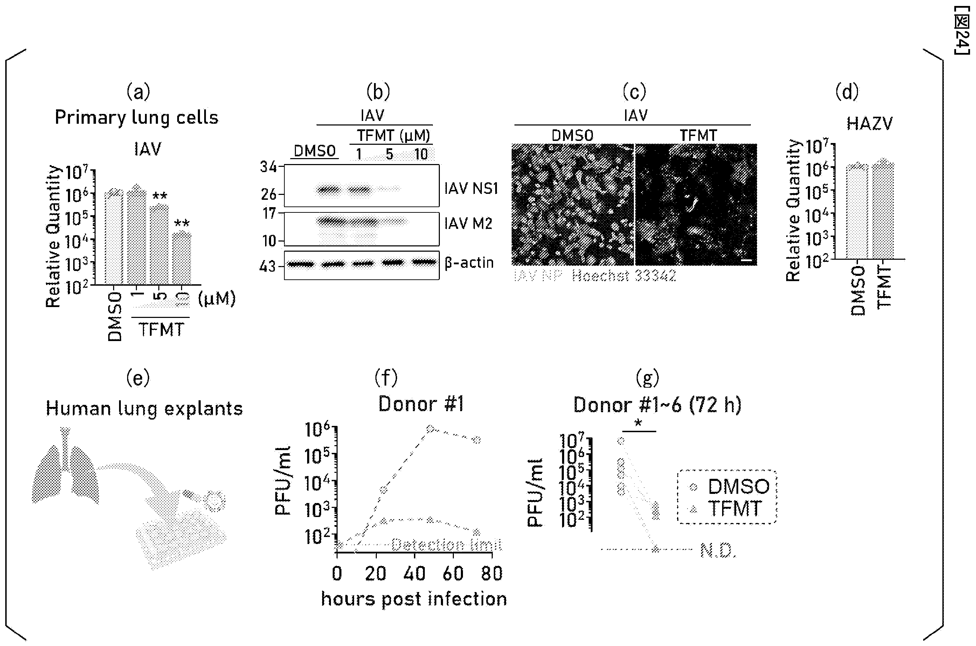

[FIG. 24] FIG. 24(a) shows a graph showing the results of qRT-PCR analysis of IAV RNA (segment 7) in normal human bronchial epithelial (NHBE) cells infected with the IAV PR8 strain at an m.o.i. of 1 for 24 hours. FIG. 24(b) shows a photographic view showing the results of Western blot analysis of indicated proteins in NHBE cells infected with the IAV PR8 strain at an m.o.i. of 1 for 24 hours. FIG. 24(c) shows a photographic view showing the results of immunostaining of NHBE cells infected with the IAV PR8 strain. FIG. 24(d) shows a graph showing the results of qRT-PCR analysis of HAZV RNA in NHBE cells infected with HAZV. FIG. 24(e) shows a schematic view of IAV infection in a human lung explant. FIG. 24(f) shows a graph showing the results of the measurement of the virus proliferation and the virus titer of seasonal IAV in the human lung tissue in one donor. FIG. 24(g) shows a graph showing the results of the measurement of the virus proliferation and the virus titer of seasonal IAV in the human lung tissue in 6 independent donors.
[FIG. 25] FIG. 25 shows a graph showing the virus proliferation of seasonal IAV in the human lung tissue.
[FIG. 26] FIG. 26 shows a photographic view showing immunohistochemistry of virus proliferation and the seasonal IAV protein in the human lung tissue.
[FIG. 27] FIG. 27 shows a graph showing the results of a cell cytotoxicity test and the level of GFP in LA-4 cells infected with IAV-GFP.
[FIG. 28] FIG. 28(a) shows a graph showing a change in body weight in a case where nasal cavity inoculation has been carried out on a mouse. FIG. 28(b) shows a schematic view of an IAV infection model in an in vivo system of a mouse. FIG. 28(c) shows a graph showing the results of qRT-PCR analysis of IAV RNA of a segment 5 in the lung tissue. FIG. 28(d) shows a graph showing the results of qRT-PCR analysis of IAV RNA of a segment 1 in the lung tissue.
[FIG. 29] FIG. 29 shows a graph showing the results of qRT-PCR analysis of IAV RNA in A549 cells infected with the IAV PR8 strain.
[FIG. 30] FIG. 30(a) shows a photographic view showing immunostaining of a viral protein of A549 cells infected with SeV or IAV. FIG. 30(b) shows a photographic view showing the results of Western blot analysis of viral proteins of A549 cells infected with SeV or IAV.
[FIG. 31] FIG. 31 shows a graph showing the results of qRT-PCR analysis of IFN-β RNA in A549 cells.
[FIG. 32] FIG. 32 shows a graph showing the results of qRT-PCR analysis of each of other RNAs in A549 cells.
[FIG. 33] FIG. 33 shows a graph showing the results of qRT-PCR analysis of each RNA in A549 cells infected with other RNA viruses.
[FIG. 34] FIG. 34(a) shows a graph showing the results of qRT-PCR analysis of each virus RNA in MTr1 KO A549 cells infected with each virus. FIG. 34(b) shows a photographic view showing the results of Western blot analysis of each protein in MTr1 KO A549 cells infected with each virus. FIG. 34(c) shows a photographic view showing the results of immunostaining of a viral protein in MTr1 KO A549 cells infected with SeV.
[FIG. 35] FIG. 35(a) shows a graph showing the results of qRT-PCR analysis of a virus and host RNA in MTr1 KO A549 cells infected with SeV. FIG. 35(b) shows a photographic view showing optical microscopic images of MTr1 KO HEK-293T cells infected with each virus.
[FIG. 36] FIG. 36(a) shows a graph showing qRT-PCR analysis of NT/U2-IAV hybrid RNA in A549 cells infected with IAV PR8. FIG. 36(b) shows a schematic view of an interaction site of N1-2'-O-Me. FIG. 36(c) shows a structure of N1 and N2 of the cap1 RNA, where N1-2'-O-Me is shown in the same manner.
[FIG. 37] FIG. 37(a) shows a schematic view of a structure of an IAV polymerase that forms a complex with RNA. FIG. 37(b) shows a schematic view showing the RNA binding region of PB2 in an enlarged manner. FIG. 37(c) shows a schematic view showing a phylogenetic tree of PB2 in influenza virus and THOV and partial sequence alignment of amino acid residues (RNA cap-binding region and conserved region).
[FIG. 38] FIG. 38(a) shows a schematic view showing a structural model of N1-m6A in RNA bound to IAV PB2. FIG. 38(b) shows a schematic view showing a structural model of N2-2'-O-Me in RNA bound to IAV PB2.
[FIG. 39] FIG. 39 shows graphs showing qRT-PCR analysis of IAV RNA of A549 cells infected with IAV PR8. FIG. 39(a) shows analysis for CAPAM KO cells, and FIG. 39(b) shows analysis for Mtr2 KO cells.
[FIG. 40] FIG. 40 shows graphs showing the results of qRT-PCR analysis of ribosomal RNA (18S) and mRNA (GAPDH) in the input and the immunoprecipitated RNA from HEK-293T cells. FIG. 40(a) shows ribosomal RNA (18S) and FIG. 40(b) shows mRNA (GAPDH). FIG. 40(c) shows a photographic view showing the results of the Western blot analysis of the PABP protein that has been subjected to the PABP immunoprecipitation.
[FIG. 41] FIG. 41(a) shows a photographic view showing the results of Western blot analysis of input and immunoprecipitated proteins in PB2-expressing WT cells and MTr1 KO HEK-293T cells. FIG. 41(b) shows a graph showing a synergy effect of BXM and TFMT.
[FIG. 42] FIG. 42(a) shows a graph showing a WST-8 assay for FIG. 41. FIG. 42(b) shows a graph showing a synergy effect of oseltamivir and TFMT. FIG. 42(c) shows the results of calculating the combination index as a scale of the synergy evaluation in FIG. 42(b)). FIG. 42(d) shows a graph showing a synergy effect of amantadine and TFMT. FIG. 42(d) shows the results of calculating the combination index as a scale of the synergy evaluation in FIG. 42(d)).
[FIG. 43] FIG. 43 shows graphs showing luciferase reporter assays of an IAV PR8 mini-replicon of Mtr1 KO HEK-293T which overexpresses each PA subunit. FIG. 43(a) shows the results of the wild type (WT), and FIG. 43(b) shows the results of the MTr1 KO cells. FIG. 43(c) shows a graph showing the virus quantity in the supernatant of MTr1 KO A549 cells infected with IAV HK483 PA I38T, which is a BXM resistant virus.
[FIG. 44] FIG. 44 shows graphs showing luciferase reporter assays of an IAV PR8 mini-replicon of MTr1 KO HEK-293T which overexpresses a PA subunit shown in the horizontal axis of each of FIG. 44(a) to FIG. 44(d).
[FIG. 45] FIG. 45 shows a view showing a CRISPR target sequence and a mutation region in the used knockout cell.

### DESCRIPTION OF EMBODIMENTS

Hereinafter, an anti-influenza virus composition according to the present invention, and a medicine, a food, a beverage, a supplement, an agricultural chemical, a feed, and a cosmetic, which use the anti-influenza virus composition according to the present invention, will be described with embodiments. However, the present invention is not limited to the following embodiments.

### (Anti-influenza virus composition)

The anti-influenza virus composition according to the present embodiment contains, as an active ingredient, tubercidin, a tubercidin derivative, or a salt thereof.

Tubercidin, a derivative thereof, and a salt thereof are known as natural organic compounds. The derivative and the salt can be appropriately selected as long as they can be used pharmaceutically.

Specifically, a compound in a case where all of R₁, R₂, and R₃ in Formula (1) described later are hydrogen atoms is known as tubercidin (CAS registration number: 69-33-0). The tubercidin derivative is a compound in which any site of tubercidin, preferably any of R₁, R₂, or R₃ in Formula (1) described later is any substituent described later.

The salt of tubercidin or a tubercidin derivative refers to a salt of any ionic substituent of tubercidin or a tubercidin derivative, and broadly refers to a pharmaceutically acceptable salt of the compound.

Examples of the pharmaceutically acceptable salt in the present specification include an acid addition salt or a base addition salt of the compound represented by Formula (1). Examples of the acid addition salt include an acid addition salt with an inorganic or organic acid (hydrochloric acid, hydrobromic acid, sulfuric acid, trifluoroacetic acid, citric acid, maleic acid, or the like). Examples of the base addition salt include a metal salt, for example, a light metal salt, and specific examples thereof include an alkali metal salt such as a sodium salt or a potassium salt, an alkaline earth metal salt such as a calcium salt, and an ammonium salt.

Through the following intensive studies, the inventors of the present invention have found tubercidin, a tubercidin derivative, or a salt thereof as an active ingredient of the anti-influenza virus composition.

That is, although it has been reported that MTr1, which is an RNA methyltransferase of a host cell, is involved in the cap snatching of the influenza virus, the details thereof have not been known.

The inventors of the present invention first produced MTr1 knockout cells and examined the replication ability of various cap snatching viruses including the influenza virus. As a result, it was found that MTr1 is essential for the replication of the A type influenza virus and the B type influenza virus. In addition, it was found that the mechanism is due to the fact that the influenza virus can snatch (steal) only a cap structure modified with MTr1. On the other hand, MTr1 was not involved in the replication of other cap snatching viruses other than the A type influenza virus and the B type influenza virus. That is, it was considered that the replication of the influenza virus can be selectively suppressed by inhibiting the function of the host MTr1.

Therefore, the inventors of the present invention further carried out screening of an existing drug library (5,597 compounds) by utilizing the three-dimensional structure information of MTr1 and a computer in order to search for an MTr1 inhibitory compound. As a result, tubercidin, a natural compound, was found as a compound that inhibits the enzyme activity of MTr1 and exhibits antiviral activity against various influenza viruses.

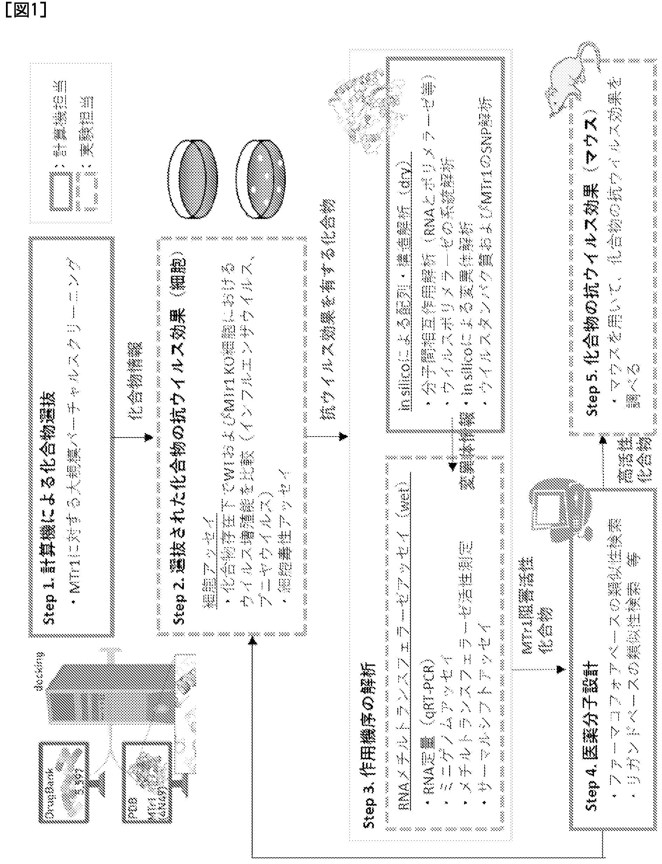

The outline of the search for the Mtr1 inhibitory compound is shown in FIG. 1.

The tubercidin derivative is preferably a compound represented by General Formula (1).

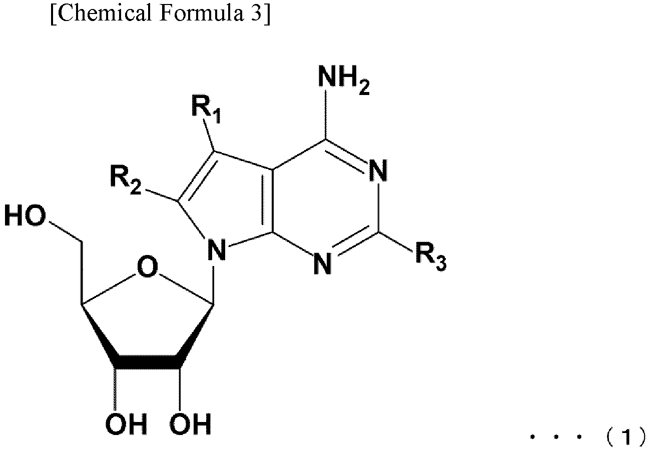

(In General Formula (1), R₁, R₂, and R₃ are each the same or different from each other, and represent a hydrogen atom, a halogen atom, an alkyl group which may have a substituent, an alkenyl group which may have a substituent, an alkynyl group which may have a substituent, an alkoxy group which may have a substituent, an amino group which may have a substituent, an amide group which may have a substituent, a cyano group, a nitro group, a hydroxy group, a sulfone group, a cycloalkyl group which may have a substituent, an aryl group which may have a substituent, an aliphatic heterocyclic group which may have a substituent, or an aromatic heterocyclic group which may have a substituent.)

R₁ and R₂ may be each the same or different from each other, and may represent a hydrogen atom, a halogen atom, an amino group which may have a substituent, an amide group which may have a substituent, or a cyano group, and R₃ may represent a hydrogen atom, an alkyl group which may have a substituent, an amino group which may have a substituent, or an aryl group which may have a substituent.

In addition, R₃ may represent a hydrogen atom.

In addition, R₁ and R₂ may be each the same or different from each other and may represent a halogen atom.

In addition, R₁ and R₂ may be each the same or different from each other and may represent an amino group which may have a substituent, or a cyano group.

In addition, R₁ may represent an alkyl group substituted with a halogen.

In addition, R₁ may represent a trifluoromethyl group.

In a case where R₁, R₂, and R₃ are a substituent containing carbon, the number of carbon atoms thereof can be optionally selected within a range acceptable for administration to a living body; however, it may be, for example, 1 to 20, may be 1 to 10, or may be 1 to 6.

The substituent which can include a linear or branched chain of carbon of an alkyl group, an alkenyl group, an alkynyl group, or an alkoxy group may be either linear or branched. In addition, the number of carbon atoms may be further 1 to 10, may be 1 to 6, or may be 1 to 3, within a range in which the structure of each substituent described above can be adopted.

The cycloalkyl group, the aryl group, the aliphatic heterocyclic group, and the aromatic heterocyclic group may consist of 1 to 3 rings or may consist of 1 or 2 rings.

Examples of the alkyl group include a methyl group, an ethyl group, an n-propyl group, an isopropyl group, a cyclopropyl group, an n-butyl group, an isobutyl group, a sec-butyl group, a tert-butyl group, an n-pentyl group, an isopentyl group, a sec-pentyl group, a tert-pentyl group, a neopentyl group, a 1-methylpentyl group, an n-hexyl group, an isohexyl group, a sec-hexyl group, a tert-hexyl group, and a neohexyl group; however, the alkyl group is not limited thereto.

Examples of the alkenyl group include a vinyl group, an allyl group, a propenyl group, an isopropenyl group, a 2-methyl-1-propenyl group, a 2-methylallyl group, and a 2-butenyl group; however, the alkenyl group is not limited thereto.

Examples of the alkynyl group include an ethynyl group, a 2-propynyl group, and a 2-butynyl group; however, the alkynyl group is not limited thereto.

Examples of the alkoxy group include a methoxy group, an ethoxy group, a propoxy group, an isopropoxyl group, a butoxy group, an isobutoxy group, a tert-butoxy group, a pentoxy group, and an isopentoxy group; however, the alkoxy group is not limited thereto.

Examples of the amino group include, for example, an amino group, an ethylamino group, a dimethylamino group, a butylamino group, a cyclopentylamino group, a 2-ethylhexylamino group, a dodecylamino group, an anilino group, a naphthylamino group, and a 2-pyridylamino group; however, the amino group is not limited thereto.

Examples of the amide group include a methylcarbonylamino group, an ethylcarbonylamino group, a dimethylcarbonylamino group, a propylcarbonylamino group, a pentylcarbonylamino group, a cyclohexylcarbonylamino group, a 2-ethylhexylcarbonylamino group, an octylcarbonylamino group, a dodecylcarbonylamino group, a phenylcarbonylamino group, and a naphthylcarbonylamino group; however, the amide group is not limited thereto.

Examples of the cycloalkyl group include a 4- to 7-membered cycloalkyl group. Specific examples thereof include, but are not limited to, a cyclobutyl group, a cyclopentyl group, a cyclohexyl group, and a cycloheptyl group.

Examples of the aryl group (aromatic hydrocarbon group) include a phenyl group, a naphthyl group, an anthryl group, an azulenyl group, an acenaphthenyl group, a fluorenyl group, a phenanthryl group, an indenyl group, a pyrenyl group, and a biphenylyl group; however, the aryl group is not limited thereto.

Examples of the aliphatic heterocyclic group include those derived from aliphatic heterocyclic rings such as an epoxy ring, an aziridine ring, a thiirane ring, an oxetane ring, an azetidine ring, a thietane ring, a tetrahydrofuran ring, a dioxolane ring, a pyrrolidine ring, a pyrazolidine ring, an imidazolidine ring, an oxazolidine ring, a tetrahydrothiophene ring, a sulfolane ring, a thiazolidine ring, an ε-caprolactone ring, an ε-caprolactam ring, a piperidine ring, a hexahydropyridazine ring, a hexahydropyrimidine ring, a piperazine ring, a morpholine ring, a tetrahydropyran ring, a 1,3-dioxane ring, a 1,4-dioxane ring, a trioxane ring, a tetrahydrothiopirane ring, a thiomorpholine ring, a thiomorpholine-1,1-dioxide ring, a pyranose ring, and a diazabicyclo[2,2,2]-octane ring; however, the aliphatic heterocyclic group is not limited thereto.

Examples of the aromatic heterocyclic group include a pyridyl group, a pyrimidinyl group, a furyl group, a pyrrolyl group, an imidazolyl group, a benzimidazolyl group, a pyrazolyl group, a pyrazinyl group, a triazolyl group, an oxazolyl group, a benzoxazolyl group, a thiazolyl group, an isoxazolyl group, an isothiazolyl group, a furazanyl group, a thienyl group, a quinolyl group, a benzofuryl group, a dibenzofuryl group, a benzothienyl group, a dibenzothienyl group, an indolyl group, a carbazolyl group, a carbolinyl group, a diazacarbazolyl group, a quinoxalinyl group, a pyridazinyl group, a triazinyl group, a quinazolinyl group, and a phthalazinyl group; however, the aromatic heterocyclic group is not limited thereto.

Examples of the halogen element include a fluorine atom, a chlorine atom, a bromine atom, and an iodine atom, and preferred examples thereof include a fluorine atom and a chlorine atom.

In a case where R₁, R₂, and R₃ further have a substituent, the substituent can be optionally selected from the organic substituents described, halogen elements, metal elements, and the like.

In a case where R₁, R₂, and R₃ have a substitution site due to a halogen element or another substitution site due to a substituent, the substitution site can also be optionally selected within a range in which the physiological function is not inhibited.

Specific examples of the substituent include the following groups described above or groups other than those described above: an alkyl group, a cycloalkyl group, an alkenyl group, an alkynyl group, an aryl group, an aliphatic heterocyclic group, an aromatic heterocyclic group, a heterocyclic group, a cycloalkoxy group (for example, a cyclopentyloxy group, a cyclohexyloxy group, or the like), an aryloxy group (for example, a phenoxy group, a naphthyloxy group, or the like), an alkylthio group (for example, a methylthio group, an ethylthio group, a propylthio group, a pentylthio group, a hexylthio group, an octylthio group, a dodecylthio group, or the like), a cycloalkylthio group (for example, a cyclopentylthio group, a cyclohexylthio group, or the like), an arylthio group (for example, a phenylthio group, a naphthylthio group, or the like), an alkoxycarbonyl group (for example, a methyloxycarbonyl group, an ethyloxycarbonyl group, a butyloxycarbonyl group, an octyloxycarbonyl group, a dodecyloxycarbonyl group, or the like), an aryloxycarbonyl group (for example, a phenyloxycarbonyl group, a naphthyloxycarbonyl group, or the like), a sulfamoyl group (for example, an aminosulfonyl group, a methylaminosulfonyl group, a dimethylaminosulfonyl group, a butylaminosulfonyl group, a hexylaminosulfonyl group, a cyclohexylaminosulfonyl group, an octylaminosulfonyl group, a dodecylaminosulfonyl group, a phenylaminosulfonyl group, a naphthylaminosulfonyl group, a 2-pyridylaminosulfonyl group, or the like), an acyl group (for example, an acetyl group, an ethylcarbonyl group, a propylcarbonyl group, a pentylcarbonyl group, a cyclohexylcarbonyl group, an octylcarbonyl group, a 2-ethylhexylcarbonyl group, a dodecylcarbonyl group, a phenylcarbonyl group, a naphthylcarbonyl group, a pyridylcarbonyl group, or the like), an acyloxy group (for example, an acetyloxy group, an ethylcarbonyloxy group, a butylcarbonyloxy group, an octylcarbonyloxy group, a dodecylcarbonyloxy group, a phenylcarbonyloxy group, or the like), an amide group, a carbamoyl group (for example, an aminocarbonyl group, a methylaminocarbonyl group, a dimethylaminocarbonyl group, a propylaminocarbonyl group, a pentylaminocarbonyl group, a cyclohexylaminocarbonyl group, an octylaminocarbonyl group, a 2-ethylhexylaminocarbonyl group, a dodecylaminocarbonyl group, a phenylaminocarbonyl group, a naphthylaminocarbonyl group, a 2-pyridylaminocarbonyl group, or the like), a ureide group (for example, a methylureide group, an ethylureide group, a pentylureide group, a cyclohexylureide group, an octylureide group, a dodecylureide group, a phenylureide group, a naphthylureide group, a 2-pyridylaminoureide group, or the like), a sulfinyl group (for example, a methylsulfinyl group, an ethylsulfinyl group, a butylsulfinyl group, a cyclohexylsulfinyl group, a 2-ethylhexylsulfinyl group, a dodecylsulfinyl group, a phenylsulfinyl group, a naphthylsulfinyl group, a 2-pyridylsulfinyl group, or the like), an alkylsulfonyl group (for example, a methylsulfonyl group, an ethylsulfonyl group, a butylsulfonyl group, a cyclohexylsulfonyl group, a 2-ethylhexylsulfonyl group, a dodecylsulfonyl group, or the like), an arylsulfonyl group or a heteroarylsulfonyl group (for example, a phenylsulfonyl group, a naphthylsulfonyl group, a 2-pyridylsulfonyl group, or the like), an amino group, a halogen atom, a fluorinated hydrocarbon group (for example, a fluoromethyl group, a trifluoromethyl group, a pentafluoroethyl group, a pentafluorophenyl group, or the like), a cyano group, a nitro group, a hydroxy group, a mercapto group, a silyl group (for example, a trimethylsilyl group, a triisopropylsilyl group, a triphenylsilyl group, a phenyldiethylsilyl group, or the like), and a phosphono group; however, the substituent is not limited thereto. Further, those in which any element of these substituents is substituted with another element or any of the above-described substituents may be used.

Any of those described above can be optionally selected from a range that does not inhibit physiological functions or a range that is allowed pharmaceutically in the administration to a living body.

That is, more specifically, the tubercidin derivative may be a compound represented by Formula (2).

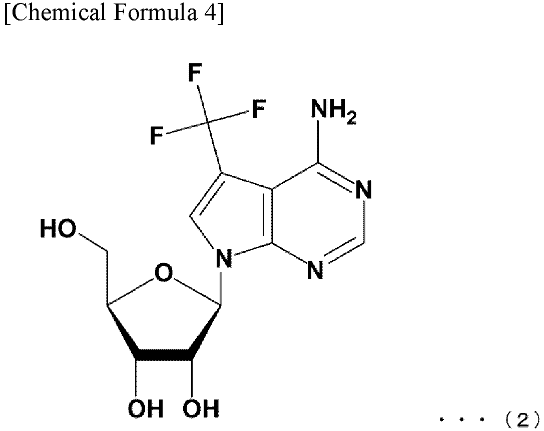

The compound represented by Formula (2), trifluoromethyl tubercidin (TFMT), is a compound having lower toxicity than tubercidin among tubercidin derivatives, and it is the most effective Mtr1 inhibitory compound that exhibits anti-influenza virus activity.

The inventors of the present invention examined 115 kinds of tubercidin derivatives and found that TFMT is the most effective component.

As a mechanism of TFMT, the inventors of the present invention expect from the analysis of the three-dimensional structure that TFMT binds to the substrate binding pocket of MTr1 and inhibits the enzyme activity. In addition, the structural analysis also predicts that the cap structure that is not modified by Mtr1 may impair the interaction with the influenza virus RNA polymerase (PB2 protein). That is, TFMT suppresses the modification function of the cap structure by binding to the host MTr1. Since the cap structure of the host mRNA that is not modified with MTr1 weakens the interaction with the RNA polymerase (PB2 protein) of the virus, it is considered that TFMT suppresses cap snatching carried out through RNA polymerase and subsequent synthesis of virus RNA.

### [Medicine, food, beverage, supplement, agricultural chemical, feed, and cosmetic]

### (Medicine)

The anti-influenza virus composition according to the present embodiment is suitably used for the treatment of influenza. That is, the anti-influenza virus composition can also be referred to as a medicine or a medicinal composition, which is used for the treatment of influenza.

In addition, other components can also be contained in the anti-influenza virus composition, whereby the anti-influenza virus composition can used as a medicine. The medicine for treating influenza may also appropriately contain various components contained in other medicines or medicinal compositions known in the related art.

The form of the medicine according to the present embodiment is not particularly limited, and it can be, for example, a dispersion body such as a solution, a sol, or a gel, or a powder. The medicine can be administered orally in the form of, for example, a tablet, a capsule, an elixir, or the like, or parenterally in the form of an enema or the like.

As the pharmaceutically acceptable carrier, in general, a carrier that is used for the pharmaceutical formulation of a medicine can be used without particular limitation. More specific examples thereof include a binding agent, an excipient, a swelling agent, and a solvent.

The medicine according to the present embodiment may contain an additive. Examples of the additive include a lubricating agent, a sweetening agent, a flavoring agent, a stabilizer, a pH buffering agent, a dissolution assisting agent, an antioxidant, and a preservative.

The medicine according to the present embodiment can be formulated by appropriately combining the above-described components and admixing them in a unit dosage form that is required for commonly accepted pharmaceutical practice.

The dose of the medicine varies depending on the kind, usage, and dosage of the drug, and the symptoms, body weight, age, sex, and the like of the patient, and thus cannot be determined unconditionally. However, the dose of the active ingredient in the anti-influenza virus composition contained in the medicine is usually may be 0.1 ng/kg to 250 mg/kg (here, kg is in terms of the body weight of the administration subject) per day, may be 0.1 ng/kg to 100 mg/kg per day, may be 1 ng/kg to 100 mg/kg per day, or may be 1 ng/kg to 50 mg/kg per day, for example, in a case of a form of administration such as injection or oral administration.

In addition, the active ingredient in the pharmaceutical formulation of the medicine may be 1% to 10% by weight, may be 1% to 7% by weight, or may be 3% to 5% by weight with respect to the total weight of the medicine.

### (Food, beverage, and supplement)

The food, the beverage, and the supplement according to the present embodiment contain the anti-influenza virus composition according to the present embodiment. The food, beverage, and supplement to be targeted are not particularly limited. Examples thereof include gel-shaped food products obtained by adding thickening polysaccharides, gelatin, or the like to the beverage according to the present embodiment and cooling the resultant mixture. Examples of the food product include processed food product.

The beverage according to the present embodiment contains the anti-influenza virus composition according to the present embodiment. Examples thereof include an extraction solution obtained by immersing the composition according to the present embodiment in water or another organic solvent, for example, alcohol such as ethanol, or another solvent and heating the resultant mixture at any temperature, or an extraction solution obtained by immersing a mixed and rolled product in a solvent such as water at any temperature. That is, the beverage according to the present embodiment can be obtained in the same manner as the drinking method for tea known in the related art. Alternatively, a method of adding the composition according to the present embodiment, which is in the form of a liquid or a solid, to any beverage (for example, a soft drink or an alcoholic beverage) can be mentioned.

### (Agricultural chemical, feed, and cosmetic)

The agricultural chemical, the feed, and the cosmetic according to the present embodiment contain the anti-influenza virus composition according to the present embodiment. The agricultural chemical, the feed, and the cosmetic can be appropriately selected in the same manner as in the above-described medicine, food product, and the like or according to the embodiments of the agricultural chemical, the feed, and the cosmetic, which are known in the related art.

### [Effect of present embodiment]

According to the present embodiment, it is possible to provide a novel anti-influenza virus agent that inhibits a mechanism of proliferation or infection of an influenza virus and thus has a target which is not a specific viral protein, where the novel anti-influenza virus agent does not cause the emergence of drug-resistant viruses. There are also provided a medicine, a food, a beverage, a supplement, an agricultural chemical, a feed, and a cosmetic, which use the anti-influenza virus agent.

In the related art, MTr1 is known as a catalyst for methylation of nucleic acids, and as a mechanism thereof, the 5' terminal of each of mRNA and small nuclear RNA (snRNA) in mammalians is capped with 7-methylguanosine (m7G) and 2,2,7-trimethylguanosine, which are bonded to RNA by a triphosphate crosslinking and are called cap0.

2'-O-ribose methyltransferase MTr1 (CMTR1/FTSJD2) is an RNA methyltransferase (MTase) that is mainly localized in the nucleus, and it catalyzes the 2'-O-methylation of the first nucleotide (N1-2'-O-Me). The cap0 mRNA and snRNA for generating a mature cap and the cap structure of the host RNA are important for stability and promoting translation. The unmethylated RNA activates an innate immune response by RIG-I-like receptors and is vulnerable to cell restriction factors such as IFIT1. The fully methylated cap1 RNA evades the innate immune mechanism. As a result, it can be said that the virus usually hijacks or mimics the cap structure of the cellular RNA.

The orthomyxovirus including the bunya virus, the influenza A virus (IAV), and the influenza B virus (IBV) depends on a strategy called cap snatching, in which the 5' terminal of the fully capped cellular RNA is cleaved and attached to the viral mRNA For example, the IAV polymerase is composed of three subunits of PA, PB1, and PB2, and the trimeric polymerase binds to the cap structure of the host RNA through PB2, initiates RNA cleavage by the PA endonuclease at 10 to 13 nucleotides downstream of the cap structure, and finally uses PB1 as a primer to synthesize the viral mRNA. On the other hand, in contrast to this, non-cap snatching viruses such as coronavirus, poxvirus, and flavivirus, which include the yellow fever virus, code their own cap-binding 2'-O-MTase, mimic the cellular cap through the 2'-O-methylation of RNA, thereby avoiding recognition by the antiviral sensor. It has been shown that the deficiency of Mtr1 in cells leads to the accumulation of host cap0 RNA in the cytoplasm and activates RIG-I and IFIT1, and has been shown that the methylation state of endogenous capped mRNA at the first nucleotide is one of the important features that prevent immune activation.

It has been reported that the Mtr1 deficiency enhances the antiviral IFN response to the influenza A virus, affects the cap snatching effect, and results in a decrease in the replication level of the influenza A virus. As a result, MTr1 is an interesting host-targeted target for an anti-influenza A virus drug and is also probably a target for drugs against other cap snatching viruses. However, the Mtr1 inhibitor has not been reported, and the possibility of the Mtr1 inhibitor as an antiviral agonist has not been investigated.

The orthomyxovirus and the bunya virus snatch the 5' cap portion of the host RNA and initiate transcription. In the present invention, it was found that the change of the cap by the host 2'-O-ribose methyltransferase MTr1 is essential for the replication of the influenza A virus and the influenza B virus; however, the change of the cap is not essential for other cap snatching viruses. Through in silico compound screening and functional analysis, it was found that trifluoromethyl-tubercidin (TFMT) inhibits MTr1 through an interaction with an S-adenosyl-L-methionine binding pocket, thereby slowing down the replication of the influenza virus. Mechanistically, TFMT impairs the binding between the host cap RNA and the virus polymerase PB2 subunit, which is the first important step in the cap snatching process.

In particular, TFMT exhibited efficacy in both human lung explant and mouse in vivo and exhibited synergistic antiviral activity with approved anti-influenza drugs such as baloxavir marboxil, oseltamivir, and amantadine.

The results shown by the inventors of the present invention have revealed an unexpected activity of MTr1 in virus replication and a conceptually new approach for inhibiting influenza virus infection.

### [Other embodiments]

The present invention has the following aspects as other embodiments.

Another aspect of the present embodiment is a compound including tubercidin, a tubercidin derivative, or a salt thereof, which is for use in the treatment or prevention of a disease associated with an influenza virus.

Still another aspect of the present embodiment is the use of a compound including tubercidin, a tubercidin derivative, or a salt thereof, which is for producing a therapeutic agent for a disease associated with an influenza virus.

Even another aspect of the present embodiment is a method for producing a therapeutic agent for a disease associated with an influenza virus, which uses a compound including tubercidin, a tubercidin derivative, or a salt thereof.

Even another aspect of the present embodiment is a method for producing a medicinal composition for treating or preventing a disease associated with an influenza virus, which uses a compound including tubercidin, a tubercidin derivative, or a salt thereof.

Even another aspect of the present embodiment is a method for producing a composition to be used for treating or preventing a disease associated with an influenza virus, which uses a compound including tubercidin, a tubercidin derivative, or a salt thereof.

Even another aspect of the present embodiment is a method for treating a disease associated with an influenza virus, which includes administering an effective amount of a compound including tubercidin, a tubercidin derivative, or a salt thereof, to a subject in need of treatment.

Yet another aspect of the present embodiment is a compound including tubercidin, a tubercidin derivative, or a salt thereof, which is for use in the treatment of a disease associated with an influenza virus.

### Examples

Hereinafter, the present invention will be described in detail according to Examples; however, the present invention is not limited to these Examples.

### [Experimental conditions and methods used]

### (Cell culture)

Human embryonic kidney (HEK)-293T cells, human alveolar adenocarcinoma A549 cells, mouse lung adenocarcinoma LA-4 cells, platinum-A (Platt-A) cells, Madin-Darby canine kidney (MDCK) cells, and Vero cells were cultured at 37°C and 5% CO₂ using a culture medium containing 10% fetal bovine serum (FBS), 100 U/ml penicillin, and 100 µg/ml streptomycin in Dulbecco's modified Eagle's medium (DMEM).

The Expi293F floating cells were cultured at 37°C and 8% CO₂ in an Expi293 expression medium (Gibco, A1435101) while shaking.

Normal human bronchial epithelial (NHBE) cells (Lonza, CC-2540) were cultured in bronchial epithelial cell growth medium (BEGM; Lonza, CC-3170).

10 µg/ml of blasticidin and 1 µg/ml of puromycin were added to the culture medium to culture the Plat-A cells.

In order to acquire an antiserum from B6 mice (#WT No. 2, 170807) infected with HAZV, C57BL/6 mice were intraperitoneally inoculated with 10⁵ plaque forming units (PFU) of the Hazara virus. The inoculation was repeated twice at an interval of 3 weeks. Three days after the last inoculation, blood was collected in a BD microtainer to obtain serum.

### (Virus)

Influenza strain A/Puerto Rico/August, 1934 (H1N1; IAV PR8) was proliferated in chicken eggs. The virus titer was quantified by a plaque assay of MDCK cells.

The encephalomyocarditis virus (EMCV) was proliferated in L929 cells, and the virus titer was quantified using L929 cells.

The Sendai virus (SeV) was proliferated in chicken eggs, and the virus titer was quantified by an HA assay using chicken erythrocytes.

A recombinant influenza strain (IAV WSN) of A/WSN/1933 (H1N1) was recovered from a pDZ ambisense plasmid 23 (donated from Adolfo Garcia Sastre). The proliferation was carried out in MDCK cells, and the titer was measured by flow cytometry using Nanobody VHH NP2 Alexa Fluor 647.

A recombinant influenza strain A/SC35M (H7N7) containing a modified NS segment (IAV SC35M-GFP) encoding NS1, the P2A site, EGFP, the T2A site, and NEP was recovered from a pHW ambisense plasmid (from Martin Schwemmle) and proliferated in MDCK cells, and the titer was measured by flow cytometry.

A recombinant vesicular stomatitis virus Indiana strain (VSV-GFP) encoding EGFP at the 5 position of the genome was infected with VACV WR vTF7.3, recovered from BSR T7/5 cells transfected with vTF7.3, and proliferated in pVSV1(+)P5_EGFP, pL, pP, pN, and BSR T7/5 cells. The virus titer was measured by a plaque assay using Vero cells.

The influenza A type virus strain A/Hong Kong/483/1997 (H5N1) (IAV HK483) and IAV HK483 I38T were proliferated and recovered in embryonated chicken eggs. The B type influenza virus strain/Hokkaido/30-4/2014 (IBV) and the influenza D virus strain D/bovine/NE/9-5/2012 (IDV) were proliferated and recovered in embryonated chicken eggs. The virus titer in the MDCK cells and the supernatant of the allantoic fluid or the cell culture product was quantified using MDCK cells at a 50% tissue culture infectious dose (TCID50).

For the Rift Valley fever virus strain MP-12, UTMB and BSR/T7-5 cells were recovered from a plasmid provided by Dr. Shinji Makino, and the Rift Valley fever virus strain MP-12 was proliferated in VeroE6 cells provided by Dr. Karl-Klaus, Conzelmann, Max von Pettenkofer-Institute of Virology.

The Hazara virus (HAZV) JC280 strain, the Issyk-Kul virus (ISKV) LEIV315K strain (accession number LC495734-6), the Soft tick bunya virus (STBV), the Av-18 strain (accession number LC495731-3), and the Dugbe virus (DUGV) strain 15AC-T25 (accession number LC579816-8) were provided by Dr. Roger Hewson (National Health Service, England), Dr. Barbara W. Johnson (Centers for Disease Control and Prevention, USA), Dr. Shuji Ando (National Institute of Infectious Diseases (NIID)), and Dr. Chang-Kweng Lim (NIID).

These viruses were proliferated in a DMEM medium containing 2% FBS, 100 U/ml penicillin, and 100 µg/ml streptomycin in Vero cells. The supernatant containing the virus was collected on the 3rd to 7th day after the inoculation. The infectious dose was titrated in SW-13 cells by a plaque formation assay in a 6-well plate.

### (Chemical agent)

BXM was purchased from BLD Pharmatech (BD00808126). Ribavirin, oseltamivir phosphate, and amantadine were purchased from Sigma-Aldrich (R9644, SML1606, 138576). The chemical substance used in the primary screening, sinefungin, and TAK-599 isavuconazonium (sulfate) were purchased from Cayman Chemical Company (13829, 23696, 23950). S-adenosyl-L-homocysteine (SAH), S-adenosyl-L-methionine (SAM), tubercidin, GDP-D-mannose disodium salt, 5-iodotubercidin (Itu), and sertindole were purchased from Sigma-Aldrich (A9384, A4377, T0642, 07508, I100, S8072). m7GpppA was purchased from New England Biolabs (S1405). Tecadenoson was purchased from BLD Pharmatech (BD00781750).

Tubercidin analog: 3-deazaadenosine (3-DZA), cladribine, clofarabine, coenzyme A, and nebularin were purchased from Cayman Chemical Company (9000785, 12085, 14125, 16147, 31329). 2-fluoroadenosine and cordycepin were purchased from Sigma-Aldrich. (656402, C3394). Vidarabine and 2'-deoxy-2-fluoroadenosine were purchased from BLD Pharmatech (BD42581, BD74284). 2-aminoadenosine was purchased from Santa Cruz Biotechnology (sc-220693A). 1-deazaadenosine, 8-azaadenosine, and fludarabine were purchased from TOCRIS (4488, 6868, 3495). Z295883282 was purchased from Enamine (Z295883282). MolPort-044-721-870 was purchased from MolPort (MolPort-044-721-870). Ganciclovir and aciclovir were purchased from Pharmeks (PHAR088857, PHAR100587). 3'-deoxy-tubercidin and 3'-deoxy-7-bromotubercidin were purchased from SHINSEI KAGAKU KOGYO CO., LTD. (manufactured on order). The compound 96 was obtained from PharmaCenter Bonn & Pharmaceutical Institute.

### (CRISPR/Cas9 knockout cell)

The sgRNA and Cas9 were introduced into a target cell by transfection or lentivirus infection, and cells were selected by using GFP or mCherry fluorescence (BD FACS Aria III) or puromycin resistance, isolated as a single clone, and checked by Western blotting and sequence analysis.

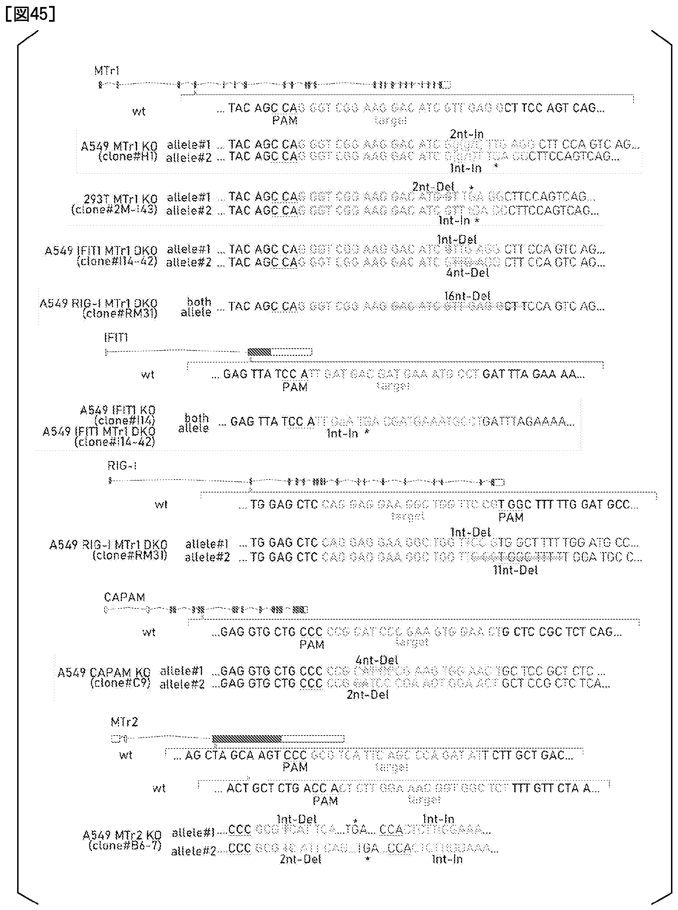

FIG. 45 shows a CRISPR target sequence and a mutation region in the used knockout cell. CRISPR targets sgRNA sequences and genotypes of all the knockout cells used in this study.

The used plasmids are shown in Tables 1 and 2.

**[Table 1]**

| Plasmid name | Purpose |
|---|---|
| pLZR-IRES-GFP | Retrovirus production (for empty control) |
| pLZR-2×Flag-hMTr1 -IRES-GFP | Retrovirus production for MTr1 expression |
| pLZR-2×Flag-hMTr1 K239A-IRES-GFP | Retrovirus production for MTr1 K239 expression |
| pLZR-IRES-mCherry | Retrovirus production for empty control |
| pLZR-Strep-Flag-hMTr1 -IRES-mCherry | Retrovirus production for MTr1 expression |
| pLZR-Strep-Flag-hMTr1 K239A-IRES-mCherry | Retrovirus production for MTr1 K239A expression |
| pCSG-BM40-TwinStrepII-hMTr1-6xHis | Recombinant MTr1 protein production |
| pEF-BOS-2×Strep-1×Flag-hMTr1 K239A | Recombinant MTr1 K239A protein production |
| pPolI-IAV seg8 Fluc (-) | IAV PR8 mini replicon reporter assay (firefly luciferase reporter) |
| pRL-SV40 | IAV PR8 mini replicon reporter assay (renilla luciferase for control) |
| pCAGGS PR8 NP | IAV PR8 mini replicon reporter assay |
| pCAGGS PR8 PA | IAV PR8 mini replicon reporter assay |

**[Table 2]**

| Plasmid name | Purpose |
|---|---|
| pCAGGS PR8 PB2-Flag | IAV PR8 mini replicon reporter assay, immunoprecipitation assay |
| pCAGGS PR8 PB2 mutant-Flag | IAV PR8 mini replicon reporter assay (PB2 Q257I, N, S, or T, PB2 I260N, Q, S or T, PB2 I261N, S, Q, or T, PB2 R264A, I, Q, K, H, E, D, V, S, P, T, W, C, Y, L, F, G, M, or N) |
| pUC-18 | IAV PR8 mini replicon reporter assay (empty plasmid) |
| pCRISPR-sghMTr1-Cas9-p2p-mCherry | CRISPR-Cas9 plasmid for hMTr1 KO cells generation |
| plentiCRISPR(sghIFIT1)-(Puro) | CRISPR-Cas9 plasmid for hIFIT1 KO cells generation |
| pSpCas9(sghRIG-I)-GFP | CRISPR-Cas9 plasmid for hRIG-I KO cells generation |
| pSpCas9(sghCAPAM)-GFP | CRISPR-Cas9 plasmid for hCAPAM KO cells generation |
| pSpCas9(sghMTr2)-GFP | CRISPR-Cas9 plasmid for hMTr2 KO cells generation |
| plentiCRISPR(sghMTr2)-(Puro) | CRISPR-Cas9 plasmid for hMTr2 KO cells generation |

### (Western blot)

The cells were dissolved in an NP40 lysis buffer (50 mM Tris-HCl pH 8.0, 150 mM NaCl, 1% NP-40) or a RIPA buffer (50 mM Tris-HCl pH 7.5, 150 mM NaCl, 1% NP-40, 0.1%). 0.1% sodium dodecyl sulfate (SDS), 0.5% deoxycholic acid, 1 mM dithiothreitol (DTT), 2 µg/ml leupeptin, 1 mM phenylmethylsulfonyl fluoride (PMSF), and 1 mM vanadate were added thereto, and the resultant mixture was incubated on ice for 10 minutes and centrifuged at a maximum speed (20,627 × g) for 15 minutes.

The supernatant was collected, mixed with a 2× sample buffer (125 mM Tris-HCl pH 6.8, 10% 2-mercaptoethanol, 4% SDS, 20% glycerol, 0.01% bromophenol blue (BPB)), and boiled at 95°C for 3 minutes. Separation was carried out by SDS-PAGE (buffer: 25 mM Tris, 192 mM glycine, 0.1% SDS), blotting was carried out onto an Immobileon-P PVDF membrane (Merck, IPVH00010) (buffer: 18.6 mM Tris, 144 mM glycine, 20% methanol), blocking was carried out with 5% skim milk (AppliChem, A0830), staining was carried out with a primary antibody at room temperature (approximately 18°C to 23°C) for 1 hour or at 4°C overnight, washing was carried out three times with TBS-T (10 mM Tris-HCl pH 8.0, 150 mM NaCl, 0.04% Tween 20), staining was carried out with a secondary antibody (HRP-bound) at room temperature for 1 hour, and washing was carried out three times with TBS-T. The bands were detected by chemiluminescence using an ECL Prime reagent (Amersham, RPN2232) and an Amersham 680 Imager.

### (Quantification of RNA)

The cells were recovered with TRIzol (Invitrogen, 15596), and RNA was extracted according to the manufacturer's protocol. The cDNA was purified using a High-Capacity cDNA Reverse Transcription Kit (Applied Biosystems, 43688). Quantitative reverse transcription polymerase chain reaction (qRT-PCR) was carried out using a Fast SYBR Green Master Mix (Applied Biosystems, 4385614) or a TaqMan Fast Advanced Master Mix (Applied Biosystems, 4444558).

Tables 3 to 5 show the PCR primers (SEQ ID NOs: 1 to 55 in the sequence listing) used and the Taq Man probes used.

In addition, Table 6 shows the product names of the antibodies used.

**[Table 3]**

| Primer or TaqMan probe name | Sequence (5' → 3'), or product information |
|---|---|
| SeV L F | GACATGGAGGGAGGAGATCA |
| SeV L R | TATAGGCTGAGCTGCCTGGT |
| IAV M(seg7) F | GGACTGCAGCGTAGACGCTT |
| IAV M(seg7) R | CATCCTGTTGTATATGAGGCCCAT |
| VSV N F | GGAGTATCGGATGCTTCCAGAACCA |
| VSV N R | ACGACCTTCTGGCACAAGAGGTT |
| EMCV VP1 F | TTATAGTGCCGGACCTGGCA |
| EMCV VP1 R | CCCAAGCTCCCAGTGTTGTC |
| IAV PR8 seg1 F ³³ | GGAGGCACAGGATGTAATCA |
| IAV PR8 seg1 R ³³ | CGTTAGTTGCGATTCCGATG |
| IAV PR8 seg2 F ³³ | ACACAAGGCCGACAGACCTA |
| IAV PR8 seg2 R ³³ | ATGCTGTTGCAGCAGGTTGG |
| IAV PR8 seg3 F ³³ | GCACTCACTTGGAAGTATGC |
| IAV PR8 seg3 R ³³ | TTGACTCGCCTTGCTCATTG |
| IAV PR8 seg4 F ³³ | CTGCTCGAAGACAGCCACAA |
| IAV PR8 seg4 R ³³ | GAGCCATCCGGCGATGTTAC |
| IAV PR8 seg5 F ³³ | ACGGCTGGTCTGACTCACAT |
| IAV PR8 seg5 R ³³ | TCCATTCCGGTGCGAACAAG |
| IAV PR8 seg6 F ³³ | TTGGTCAGCAAGTGCATGTC |
| IAV PR8 seg6 R ³³ | ACAGCCACTGCTCCATTATC |

**[Table 4]**

| Primer or TaqMan probe name | Sequence (5' → 3'), or product information |
|---|---|
| IAV PR8 seg7 F ³³ | CAAGCAGCAGAGGCCATGGA |
| IAV PR8 seg7 R ³³ | GACCAGCACTGGAGCTAGGA |
| IAV PR8 seg8 F ³³ | AGCAGATAGTGGAGCGGATT |
| IAV PR8 seg8 R ³³ | GTACAGAGGCCATGGTCATT |
| vRNAtag RT ³⁴ | GGCCGTCATGGTGGCGAATGAATGGACGGA GAACAAGGATTGC |
| mRNAtag RT ³⁴ | CCAGATCGTTCGAGTCGTTTTTTTTTTTTTTT TTCTTTAATTGTC |
| cRNAtag RT ³⁴ | GCTAGCTTCAGCTAGGCATCAGTAGAAACAA GGGTATTTTCTTT |
| vRNAtag ³⁴ | GGCCGTCATGGTGGCGAAT |
| vRNA R ³⁴ | CTCAATATGAGTGCAGACCGTGCT |
| mRNAcRNA F ³⁴ | CGATCGTGCCCTCCTTTG |
| mRNAtag ³⁴ | CCAGATCGTTCGAGTCGT |
| cRNAtag ³⁴ | GCTAGCTTCAGCTAGGCATC |
| GFP F | GACGTAAACGGCCACAAGTT |
| GFP R | GAACTTCAGGGTCAGCTTGC |
| cap qPCR NT-seg1 F | CAGGTAAGTATGCAAAAG |
| cap qPCR U2-seg1 F | ATCGCTTCTCGGCAAAAG |
| cap qPCR seg1 R | GTATTTCATTGCCATCATCC |
| U2 F | TTTTGGAGCAGGGAGATGGAA |
| U2 R | GGTACTGCAATACCAGGTCGA |

**[Table 5]**

| Primer or TaqMan probe name | Sequence (5' → 3'), or product information |
|---|---|
| GAPDH F | AAGGTGAAGGTCGGAGTCAA |
| GAPDH R | AATGAAGGGGTCATTGATGG |
| IBV M(seg7) F | ATGTCGCTGTTTGGAGACACA |
| IBV M(seg7) R | TTCTTTCCCACCGAACCAACA |
| THOV seg5 F | CTTAAGAACAGCAGGCCCAG |
| THOV seg5 R | CAGGGGATTCTAAGGGCTTC |
| HAZV NP F | TGGAGAAAGGATGTCGGCTT |
| HAZV NP R | ATTGATCACTGTCCCTGGCA |
| STBV L F | CAGGACAAGATGCTGACGAA |
| STBV L R | GACTTTGGGGTGTTTGTGCT |
| ISKV NP F | CCTTGGATGTGGGCAAGAGG |
| ISKV NP R | TAGCAGGAATAGCCCCGAAG |
| DUGV NP F | GCAGAGTACAAAGTGCCTGG |
| DUGV NP R | CAGCATTTTCTCCGTCTCCG |
| T7 promoter F | TAATACGACTCACTATA |
| T7-GGG-N25 R | CACTTTCACTTCTCCCTTTCAGTTTCCCTATA GTGAGTCGTATTA |
| 18S (Eukaryotic 18S rRNA) | Hs99999901_s1 |
| hIFNB 1 | Hs01077958_s1 |
| hFTSJD2 (MTr1) | Hs00299397_m1 |
| hMxA | Hs00895608_m1 |
| hISG15 | Hs01921425_s1 |
| hIFIT1 (ISG56) | Hs03027069_s1 |

**[Table 6]**

| Antibody name | Product information |
|---|---|
| IAV NS1 mAb (NS1-23-1) | Santa Cruz, sc-130568 |
| IAV M2 mAb (14C2) | Santa Cruz, sc-32238 |
| IAV NP mAb (5D8) | Thermo Fisher, HYB-156-01-02 |
| IAV NP VHH NP2-AF647 | VHH-LPETG-His was produced in E. coli and labeled by sortase reaction with GGGK-AF647²⁴ |
| IAV PB2 pAb | Novus bio, NBP2-42878 |
| Sendai virus pAb | MBL, PD029 |
| VSV N mAb (10G4) | Kerafast, EB0009 |
| HAZV anti-serum (#WT No. 2, 170807) | Anti-serum from HAZV-infected B6 mouse |
| IBV NP mAb (1A2A11) | Thermo Fisher, MA515375 |
| MTr1 pAb | Novus bio, NBP1-83047 |
| IFIT1 (D2X9Z) mAb | CST, 14769 |
| RIG-I (D-12) mAb | Santa Cruz, sc-376845 |
| PABP pAb | Abcam, ab21060 |
| Rabbit IgG mAb | Abcam, ab172730 |
| GFP (B-2) mAb | Santa Cruz, sc-9996 |
| β-actin mAb (13E5)-HRP | CST, 5125 |
| GAPDH mAb (14C10)-HRP | CST, 3683 |

### (Fluorescence quantification)

The GFP fluorescence of the IAV SC35-GFP-infected cells was quantified using FIJI (ImageJ). The threshold value of the 8-bit image acquired with a Cell-IQ fluorescence microscope was adjusted, and the fluorescence was quantified with "Analyze Particles". The relative "IntDen" value was calculated as a relative fluorescence unit/region of interest (RFU/ROI).

### (Retrovirus production)

The semi-confluent Plat-A cells in the 6-well plate were transfected with 2 µg of a transfection mix or a retrovirus vector (pLZR backbone), 1 µg of pCMV-VSV-G-RSV-ReV, and 500 µl of PEI MAX as well as 10 µg Opti-MEM (Thermo Fischer Scientific, 31985). After 6 hours, the culture medium was replaced with a fresh culture medium. Two days after the transfection, the supernatant was collected, passed through a 0.45 µm pore filter (Sigma-Aldrich, WHA10462100), and used for infecting target cells with 10 µg/ml of Polybrene (Merck, TR-1003-G).

### (Virus titer)

The IAV PR8 titer (PFU/ml) was determined by a plaque assay using MDCK cells. MDCK cells were seeded in a multi-well plate one day before the infection. A 1:10 stage diluted liquid of the supernatant containing IAV was added to the well and incubated at 37°C for 1 hour. One hour after the infection, the cells were washed twice with DMEM medium, and then an overlay medium (DMEM, 1.5% Avicel PH-101 (Sigma-Aldrich, 11365), 0.2% bovine serum albumin (BSA; Biomol, 01400.100), 100 U/ml penicillin, 100 µg/ml streptomycin, and 0.5 µg/ml TPCK-treated trypsin (Thermo Fischer Scientific, 20233)) was added thereto. Three days after the infection, the cells were washed with phosphate buffered saline (PBS) and fixed with 6% PBS buffered formaldehyde. The fixed cells were stained with a 0.5% crystal violet solution. The cells were washed with water, and then the plaques were counted to calculate the virus titer. The titers (tissue culture infectious dose median value (TCID50)/ml) of IBV, IAV HK483, and RVFV were each measured using MDCK and Vero cells.

### (IAV mini-replicon reporter assay)

The assay was carried out using the method described in Cell Host Microbe 17, 309-319. HEK-293T WT cells or MTr1 KO cells were transfected with IAV PB2, PB1, PA, NP, a firefly luciferase mini-genome construct (negative sense), and an expression plasmid of Renilla luciferase (ratio = 1:1:0.1:1:1:0.1). As a negative control, an expression plasmid obtained by replacing a PB2 or PA expression plasmid with a blank expression plasmid was used. One day after the transfection, a Dual-Luciferase Reporter System (Promega, E1980) equipped with a Tristar2 LB942 illuminometer (Berthold Technologies) was used to measure luciferase activity. The firefly luciferase activity was normalized with respect to the Renilla luciferase activity.

### (Flow cytometry analysis)

The detached cells were fixed with a 4% paraformaldehyde PBS solution for 10 minutes, washed twice with PBS, subjected to a permeation treatment with 0.05% Triton X-100, washed twice with PBS containing 2% FBS, and blocked with 0.05% Tween 20 PBS (PBS-T) and 5 mg/ml BSA for 30 minutes. The cells were stained with an anti-NP nanobody at room temperature for 1 hour and washed three times with PBS containing 2% FBS, and the NP-expressing cells were measured with Canto II (BD) and analyzed using FlowJo 10.6.2 software (Tree Star).

### (Cell cytotoxicity assay)

In order to evaluate the cell survival rate, WST-8 (CCK8) tetrazolium salt (Abcam, ab228554) was used according to the manufacturer's instructions.

### (Immunostaining)

The cells were fixed with a 4% paraformaldehyde solution in PBS for 10 minutes, washed twice with PBS, subjected to a permeation treatment with 0.05% Triton X-100, washed twice with PBST, blocked with 5 mg/ml BSA in PBST for 30 minutes, and subjected to primary staining. The antibody was washed in 5 mg/ml BSA at room temperature for 1 hour, the cells were washed three times with PBS-T for 10 minutes each time, stained with a secondary antibody in 5 mg/ml BSA for 30 minutes, washed once in PBS containing 1 µg/ml Hoechst 33342 (Thermo Fischer Scientific, 62249) for 10 minutes, and washed twice in PBS-T for 10 minutes each time.

### (Fluorescence microscope)

The fluorescence microscopic image was acquired using InCellis (CENiBRA) equipped with a 10× objective lens, a Cell-IQ fluorescence microscope (CM Technologies) equipped with a 4× objective lens, or an SP8 confocal microscope (Leica) equipped with a 10×/0.30HC PL FLUOTAR DRY objective lens and a HyD detector.

### (Creation of phylogenetic tree)

The phylogenetic tree was generated with MEGA X software by using the neighbor-joining method.Phylogenesis was evaluated using a bootstrap method (1,000 times of replications). The references for the PB2 sequences are as follows: IAV PR8 (GenBank: NC_002023.1), IAV WSN (GenBank: LC333182.1), IAV SC35M (GenBank: DQ266097.1), IAV HK483 (GenBank: AF258839.1), IBV (GenBank: AF101982.1), ICV (GenBank: AF170576.2), IDV (GenBank: LC318665.1), and THOV (GenBank: NC_006508.1). The references for the bunya virus S segment sequences are as follows: RVFV (GenBank: KU925457.1), Crimean-Congo hemorrhagic fever virus (CCHFV) (GenBank: M86625.1), HAZV (NCBI: NC_038711.1), ISKV (GenBank: LC495736.1), STBV (GenBank: LC495733.1), and DUGV (GenBank: KU925457.1).

### (Screening of Mtr1 inhibitor based on structure)

The structure-based virtual screening of the MTr1 inhibitor was carried out using molecular docking to 5,597 physiologically active compounds having a molecular weight of 200 Da to 800 Da, which are present in the DrugBank database. The docking simulation was carried out using a Glide SP docking program (Schredinger, LLC) by using a grid box defined by the SAM binding pocket of the crystal structure of human Mtr1 (PDB ID: 4N49). From the results thereof, only the drugs approved by the FDA were selected, and then the top 30 compounds were selected for in vitro research.

### (TFMT-Mtr1 docking)

The docking simulation was carried out using a Glide SP docking program (Schredinger, LLC) by using a grid box defined by the SAM binding pocket of the crystal structure of human Mtr1 (PDB ID: 4N49). The docking pose having the highest docking score was selected.

### (Production and purification of recombinant protein)

The recombinant human MTr1 WT protein and K239A protein were expressed in Expi293F cells using an ExpiFectamine Transfection Kit (Gibco, A14524) and purified using an affinity tag (Twin Strep-tag II). The cell pellet was dissolved in 50 mM Tris-HCl (pH 8.0), 150 mM NaCl, 1% NP-40, 1 mM DTT, 2 µg/ml leupeptin, 1 mM PMSF, and 1 mM vanadate, and crushed by three freeze-thaw cycles (from liquid nitrogen to a water bath at 37°C). After removing cell debris by centrifugation, the supernatant was mixed with Strep-Tactin Superflow Agarose (Merck, 71592-4) in an open column (Pierce, 29920), washed, and eluted with a buffer solution containing biotin (Merck, 71613-3, [100 mM Tris-HCl pH 8.0, 150 mM NaCl, 1 mM ethylenediaminetetraacetic acid (EDTA), 2.5 mM desthiobiotin, pH 8.0]). The protein concentration was determined by creating a standard curve using a BSA standard protein (Pierce, 23210) by SDS-PAGE accompanied by Coomassie blue staining.

### (In vitro RNA transcription)

The Cap0 RNA was synthesized according to the manufacturer's instructions by using a HiScribe T7 Quick High Yield RNA Synthesis Kit (NEB, E2050S) and a cap analog, 3'-O-Me-m7G(5')-ppp(5')G (NEB, S1411). The annealed T7 promoter-GGG-N25 DNA was used as a template (shown in the primer table in Table 5). The synthesized RNA, 3'-O-Me-m7GpppGpG (pN27), was used in a methyltransferase activity assay of human MT r1.

### (Methyltransferase activity assay)

A reaction buffer (50 mM Tris-HCl pH 8.0, 5 mM KCl, 1 mM MgCl₂, 1 mM DTT), methyltransferases (0.8 µM human MTr1 or 0.8 µM human MTr1 K239A), an inhibitor of interest, 10 µM 3'-O-Me-m7GpppGpG (pN27) cap0 RNA, and 1.2 µM (0.02 µCi/µl) adenosyl-L-methionine, as well as S-[methyl-³H] (SAM[³H]) (PerkinElmer, NET155V250UC) were incubated at 37°C overnight. The reaction sample was purified using a mini quick oligo column (Roche, 11814397001), and free SAM [3H] was removed. The purified sample was diluted with ULTIMA GOLD (PerkinElmer, 6013329), and the methyltransferase activity was measured in terms of the number of disintegrations per minute (DPM) by using a scintillation counter LS6500 (Beckman Coulter).

### (Thermal shift assay)

The thermal stability of the protein was measured using a SYPRO Orange dye (Sigma, S5692). The purchased stock solution (5,000 times) was diluted 50 times with a thermal shift assay buffer solution (25 mM HEPES pH 7.5, 150 mM NaCl) before use. The reaction mixture was prepared by mixing 5 µl of the diluted SYPRO Orange dye, 1 µg of protein, and a compound having each concentration in a total amount of 50 µl of the thermal shift assay buffer solution. The thermal stability was analyzed with a Step One Plus real-time PCR system equipped with a TAMRA dye detection setting. The measured values were normalized using GraphPad Prism software.

### (Human lung explant)

The IAV infection in the human lung explant was carried out as described in Emerg Microbes Infect 8, 1763-1776. About 100 mg/well (12-well plate) of the human lung tissue blocks without tumors, which had been obtained from a patient who had undergone lung surgery at the Muenster University Hospital, was used. Incubation was carried out overnight at 33°C and 5% CO₂ in a Roswell Park Memorial Institute 1640 medium (RPMI) containing 2 mM L-glutamine, 100 U/ml penicillin, 100 µg/ml streptomycin, and 0.1% BSA. 1 hour before the IAV infection, 10 µM TFMT was added thereto. A culture medium containing IAV was injected into a lung block, the tissue was washed after 1 hour of infection, and the tissue was placed in a fresh culture medium containing 10 µM TFMT. The supernatant was collected for the virus titer measurement by the plaque assay, and the lung tissue was immunostained using an anti-IAV NP antibody.

It is noted that all patients provided written consent to provide lung tissue for scientific purposes. The ethical approval was provided by the ethics council of Arztekammer Westphalen-Lippe (AZ: 2016-265-f-S).

### (Synergistic effect)

The evaluation of the synergistic effect was carried out as described in Pharmacol. Rev. 58, 621-681.

### (Statistical information)

The statistics were calculated using a two-sided Student's t-test for the cell line assay, a Mann-Whitney test for the in vivo assay in mice, and a Wilcoxon matched-pairs signed-rank test for the human lung explant assay (*p < 0.05, **p < 0.01, ***p < 0.001).

### [Test Example 1: Mtr1-dependent cap structure modification]

In order to establish a functional antiviral screening system for an MTr1 inhibitor, first, it was confirmed that MTr1 is required for the replication of various cap snatching viruses.

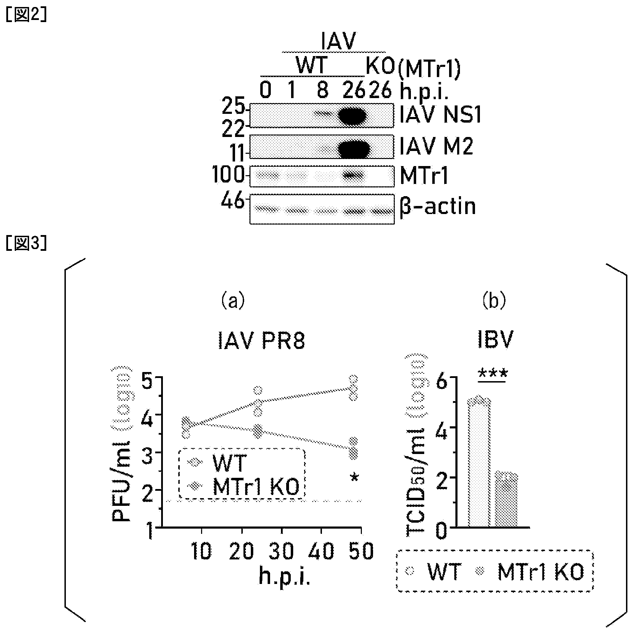

FIG. 2 shows a photographic view of Western blot showing the generation of MTr1 knockout (KO) A549 cells by CRISPR/Cas9. In the figure, wild type (WT) cells and MTr1 KO A549 cells indicate cells infected with influenza A virus (IAV) PR8 (m.o.i., 1) (m.o.i.: multiplicity of infection).

These cells were infected with two kinds of orthomyxoviruses, IAV (PR8 or H1N1), and IBV (influenza B virus) (Victoria), and the replication level was monitored.

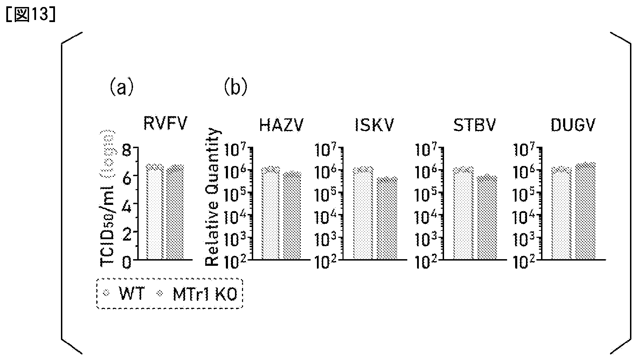

FIG. 3 shows graphs showing accumulation of IAV and IBV viruses in wild type and MTr1 KO A549 cells. FIG. 3(a) shows the virus proliferation of IAV (H1N1 or PR8) in WT and MTr1 KO A549 cells. After infection (m.o.i., 1), the supernatant was collected at a designated moment after infection, and the virus titer was measured by a plaque assay. The detection limit is indicated by a dotted line at the lower part. FIG. 13(b) shows the virus quantity in the supernatant from WT and MTr1 KO A549 cells, which have been infected with IBV at a m.o.i. of 0.1 for 48 hours.

From these results, it is worthy of notice that the accumulation of IAV and IBV was significantly impaired in the Mtr1 KO A549 cells.

FIG. 4(a) shows a graph showing qRT-PCR analysis of IAV RNA (segment 7) in WT and MTr1 KO A549 cells, which have been infected with IAV PR8 (m.o.i., 1).

FIG. 4(b) shows a graph showing qRT-PCR analysis of individual IAV RNA segments in WT and MTr1 KO A549 cells, which have been infected with IAV PR8 for 24 hours at an m.o.i. of 1.

FIG. 4(c) shows a photographic view showing immunostaining of a viral protein in WT and MTr1 KO A549 cells, which have been infected with IAV PR8 (m.o.i., 10) for 19 hours.

FIG. 4(d) shows a graph showing qRT-PCR analysis of IBV RNA in WT and MTr1 KO A549 cells, which have been infected with IBV for 24 hours at an m.o.i of 1.

FIG. 4(e) shows a photographic view showing Western blot analysis of the indicated proteins in WT A549 cells or MTr1 KO A549 cells infected with IBV for 24 hours at an m.o.i. of 1.

According to these results, in the MTr1 KO cells, the disappearance of the virus RNA level and the expression of the viral protein were clearly observed in conjunction with the above-described results.

24 hours before the IAV infection, the cells were infected with a retrovirus expressing MTr1 or MTr1 K239A or with a virus expressing none of them.

FIG. 4(f) shows a photographic view showing Western blot analysis of each protein in WT and MTr1 KO A549 cells, which have been infected with the IAV PR8 strain for 24 hours at an m.o.i of 1.

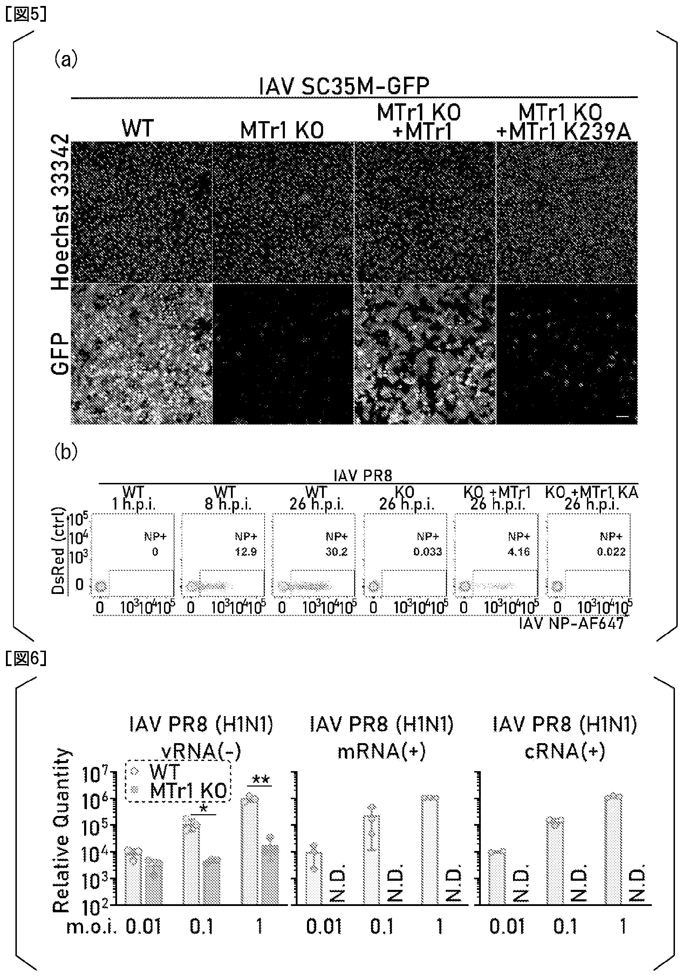

FIG. 5(a) shows a photographic view showing each immunostaining in WT and MTr1 KO A549 cells, which have been infected with the IAV PR8 strain for 24 hours at an m.o.i of 1.

FIG. 5(b) shows graphs showing the results of flow cytometry in WT and MTr1 KO A549 cells, which have been infected with the IAV PR8 strain for 24 hours at an m.o.i of 1.

As shown in these results, the IAV replication in the MTr1 KO cells was restored by the re-expression of MTr1 but was not restored by the re-expression of MTr1 K239A, which is a catalytically inactive mutant.

FIG. 6 shows graphs showing the chain-specific qRT-PCR analysis of IAV vRNA, mRNA, and cRNA (segment 5) in WT and MTr1 KO A549 cells, which have been infected with IAV PR8 for 24 hours at each m.o.i. shown in the figure.

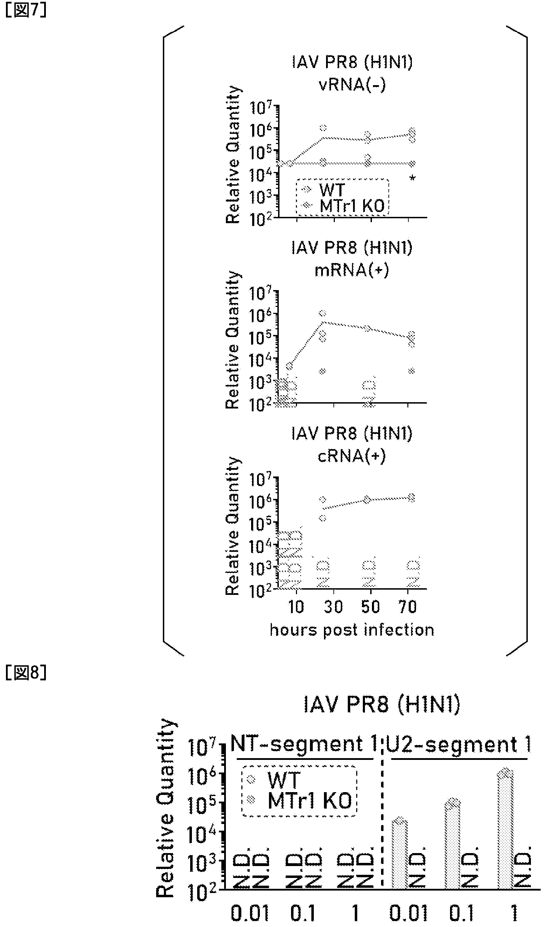

FIG. 7 shows graphs showing the chain-specific qRT-PCR analysis of IAV vRNA, mRNA, and cRNA (segment 5) in WT and MTr1 KO A549 cells, which have been infected with IAV PR8 for 24 hours at each m.o.i. and each time shown in the figure.

According to the strand-specific quantitative reverse transcription polymerase chain reaction (qRT-PCR), only IAV vRNA was detected in the MTr1 KO cells, and mRNA and cRNA were not detected.

In conjunction with the findings in the related art, a U2 segment 1 (PB2) hybrid primer was used to quantify, by qRT-PCR, the level of IAV mRNA (segment 1) that had been specifically snatched from the U2 spliceosome snRNA, whereby the defect in the cap snatching step of IAV replication in the MTr1 KO cells was examined.

FIG. 8 shows a graph showing qRT-PCR analysis of NT/U2-IAV hybrid RNA in WT and MTr1 KO A549 cells, which have been infected with IAV PR8 for 24 hours at each m.o.i. shown in the figure.

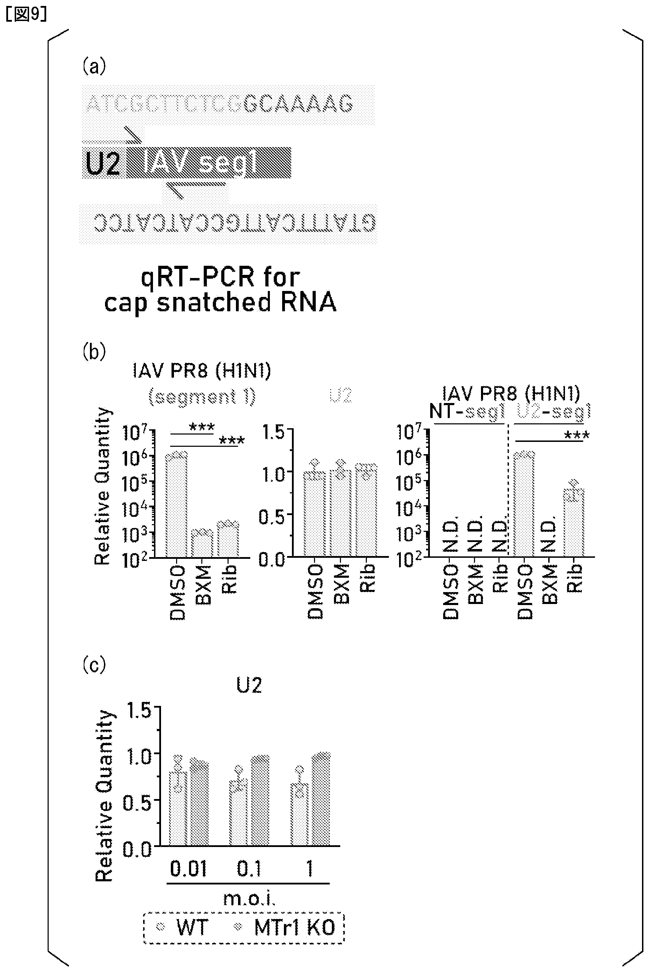

FIG. 9(a) shows a schematic view showing primer sequences for cap snatching-specific qPCR.

FIG. 9(b) shows graphs showing qRT-PCR analysis of IAV RNA (segment 1), U2, and U2-IAV RNA (segment 1) hybrid RNA (cap snatching RNA) in A549 cells infected with IAV PR8 for 24 hours at an m.o.i 1. 0.1 µM of BXM or 100 µM of ribavirin (Rib) was added 3 hours before the infection. A non-targeting (NT)-segment 1 hybrid primer was used as a control.

FIG. 9(c) shows a graph showing the qRT-PCR analysis of the U2 RNA of the same sample as in FIG. 8.

In order to examine whether MTr1 is required only for the PR8 strain (H1N1) of IAV or whether other orthomyxoviruses also depend on this host factor in the same manner, the replication efficiency of various IAV strains and other viruses belonging to orthomyxovirus families was also examined.

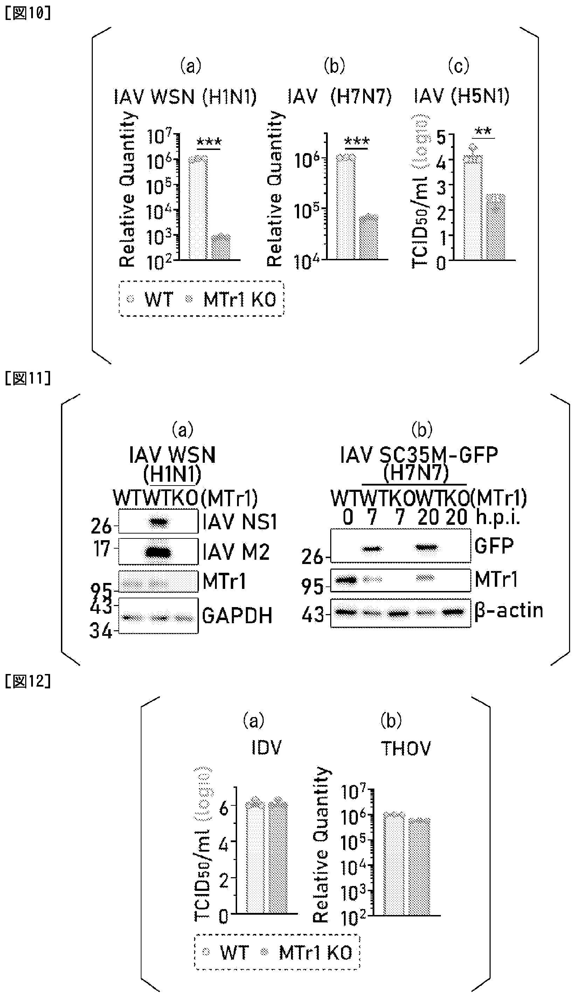

FIG. 10 shows graphs showing qRT-PCR analysis and virus quantity of WT and MTr1 KO cells, which have been infected with IAV. The graphs show the qRT-PCR analysis of IAV RNA (segment 7) or GFP RNA (inserted into segment 8) in WT and MTr1 KO A549 cells, which have been infected with IAV WSN (FIG. 10(a)) or IAV SC35M-GFP (H7N7) (FIG. 10(b)) at 1 particle/1 cell for 24 hours. FIG. 13(c) shows the virus quantity in the supernatant of WT and MTr1 KO A549 cells, which have been infected with IAV HK483 (H5N1) at an m.o.i. of 0.1 for 48 hours.

FIG. 11 shows a photographic view showing the results of Western blotting of WT and MTr1 KO cells, which have been infected with IAV. FIG. 11(a) shows the results in WT and MTr1 KO A549 cells, which have been infected with IAV WSN at 1 particle/1 cell for 24 hours, and FIG. 11(b) shows the results in WT and MTr1 KO A549 cells, which have been infected with SC35M-GFP at 0.1 infectious particles/cell for the indicated time.

From the results in the figure, not only PR8 (H1N1) but also WSN (H1N1), SC35M (H7N7), as well as high pathogenic avian IAV HK483 (H5N1) exhibited a replication defect in the MTr1 KO cells.

FIG. 12 shows graphs showing qRT-PCR analysis and virus quantity of WT and MTr1 KO cells, which have been infected with IDV or THOV. FIG. 12(a) shows the virus quantity in the supernatant of WT and MTr1 KO A549 cells, which have been infected with IDV at an m.o.i. of 0.1 for 48 hours. FIG. 12(b) shows the results of qRT-PCR analysis of THOV RNA in WT and MTr1 KO A549 cells, which have been infected with THOV for 24 hours at an m.o.i. 1.

As shown in the figure, the MT r1 deficiency had a minimal effect on the production of virus particles and the RNA replication of the other two orthomyxoviruses, the influenza D virus (IDV), and the Thogoto virus (THOV), or had no effect at all, which suggested that the demand for MT r1 is different between the orthomyxoviruses.

Next, it was examined whether or not MTr1 functions as a general host factor of the cap snatching virus. In addition to the orthomyxovirus, it is well known that the bunya virus also has a cap snatching activity. Accordingly, various bunya viruses (STBV) including Rift Valley fever virus (RVFV), Hazara virus (HAZV), Issyk-Kul virus (ISKV), and Soft tick, and Dugbe virus (DUGV) were examined.

FIG. 13 shows graphs showing virus quantity and RNA amount in WT and MTr1 KO A549 cells, which have been infected with various viruses. FIG. 13(a) shows the virus quantity in the supernatant from WT and MTr1 KO A549 cells, which have been infected with RVFV at a m.o.i. of 0.1 for 2 days. FIG. 13(a) shows the results of qRT-PCR analysis of the indicated virus RNA in WT and MTr1 KO A549 cells, which have been infected with HAZV (m.o.i., 0.1), ISKV (m.o.i., 0.01), STBV (m.o.i., 0.1), or DUGV (m.o.i., 0.1) for 24 hours.

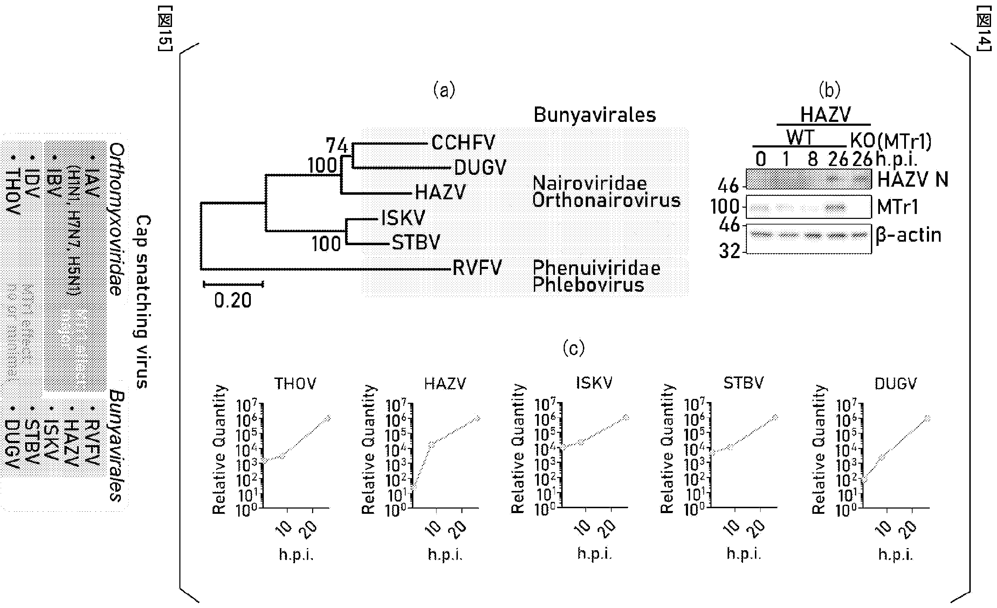

FIG. 14(a) shows a schematic view showing a phylogenetic tree of an S segment of the bunya virus.

FIG. 14(b) shows a photographic view showing the results of Western blot analysis of the indicated proteins in WT A549 cells or MTr1 KO A549 cells, which have been infected with HAZV at the time shown in the figure at an m.o.i. of 0.1.

FIG. 14(c) shows a graph showing the results of qRT-PCR analysis of the designated virus RNA in A549 cells at the time and each m.o.i. shown in the figure. m.o.i. is each 1 (THOV), 0.1 (HAZV), 0.01 (ISKV), 0.1 (STBV), or 0.1 (DUGV). The statistic was calculated using a two-sided Student's t-test (*p < 0.05, ***p < 0.001).

As shown in the figure, the differences in the RNA replication level and the virus quantity in the supernatant of the MTr1 KO cells infected with these bunya viruses were very small or not observed as compared with those in the wild type (WT) cells. These data suggest that the cap snatching activities of these bunya viruses and two orthomyxoviruses IDV and THOV are independent of the Mtr1-dependent cap modification.

FIG. 15 shows a schematic view showing an outline of the dependence of Mtr1 on the replication of the cap snatching virus.

Each of the above data indicates that the host-specific inhibitor for MTr1 specifically inhibits IAV and IBV among the cap snatching viruses regardless of the subtype or strain.

### [Test Example 2: Verification of MTr1 inhibition of TFMT by in silico compound screening]

Considering the specific and strong restriction of IAV and IBV replication due to the MTr1 deficiency, MTr1 is an attractive target for anti-IAV drugs and anti-IBV drugs, and it is considered to be ideally effective against high pathogenic avian IAV strains such as H5N1. It is known that MTr1 transfers a methyl group from S-adenosyl-L-methionine (SAM), which is a methyl donor, to a cap0 RNA receptor, and generates cap1 RNA and S-adenosyl-L-homocysteine (SAH) as by-products. Therefore, a compound that specifically binds to the SAM binding pocket of MTr1 may act as an inhibitor.

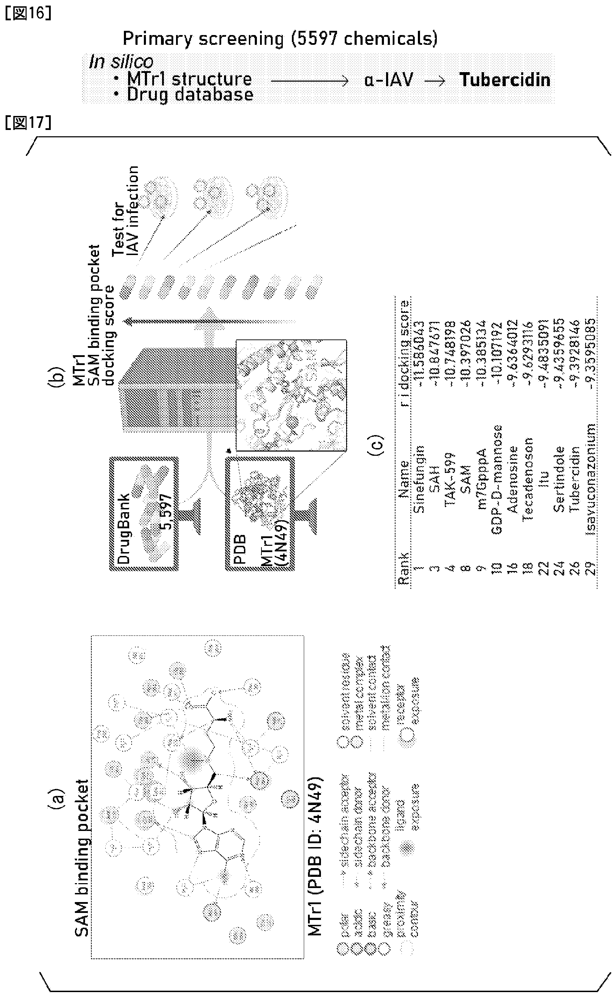

FIG. 16 shows a schematic view showing a schematic flowchart of primary screening.

FIG. 17(a) shows a schematic view showing a 2D interaction between SAM and human MTrl (PDB ID: 4N49). The image was generated using MOE software.

FIG. 17(b) shows a schematic view of a screening strategy for acquiring an Mtr1 inhibitor candidate. The MTr1 structure of PDB was used to create (ID: 4N49) at BioRender.com.

5,597 compounds of DrugBank were docked to the SAM binding pocket of the crystal structure of human MTr1.

FIG. 17(c) shows a schematic view showing docking scores and virtual screening rankings of representative compounds.

As shown in the figure, the compounds were listed in the order of the calculated affinity score, and it was confirmed that SAM, SAH, and SAM-analog sinefungin are ranked in the top 10 high affinity binders by the in silico screening method.

Next, the anti-IAV activity of 12 commercially available compounds having a high affinity score among the top 30 was examined.

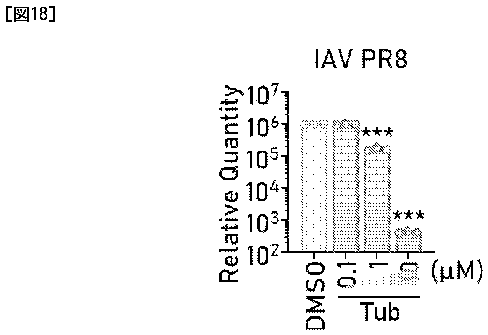

FIG. 18 shows a graph showing the results of qRT-PCR analysis of IAV RNA (segment 7) in A549 cells infected with the IAV PR8 strain at an m.o.i. of 1 for 24 hours. Tubercidin at the indicated concentration was added 3 hours before the infection.

FIG. 19(a) shows a graph showing the results of quantification of the level of GFP in A549 cells infected with the IAV-GFP SC35M strain (m.o.i., 0.1) for 19 hours. 100 µM of each compound was added 3 hours before the infection.

FIG. 19(b) shows a graph showing the results of another quantification of the level of GFP in A549 cells infected with the IAV-GFP SC35M strain (m.o.i., 0.1) for 19 hours. Each compound was added at 0.1, 1, 10, or 100 µM 3 hours before the infection.

FIG. 19(c) shows a graph showing the results of the quantification of the GFP level and the cell cytotoxicity test (WST-8) in A549 cells infected with IAV-GFP (m.o.i., 0.1) for 19 hours. Tubercidin was added at the concentration shown in the figure 3 hours before the infection.

Interestingly, tubercidin, which is an adenosine analog, was identified as the most effective compound, and it was confirmed that the inhibition depended on the dose at 0.1 to 100 µM and confirmed that the toxicity was slight.

FIG. 19(d) shows a graph showing the results of qRT-PCR analysis of IAV RNA (segment 7) in A549 cells infected with the IAV PR8 strain at an m.o.i. of 1 for 24 hours.

Tubercidin having the concentration indicated in the figure was added 3 hours before the infection.

As shown in the figure, tubercidin also had an effect on the IBV infectious disease.

FIG. 20 shows a graph showing the results of a thermal shift assay of recombinant MTr1 using SYPRO Orange. The assay was carried out at the concentration indicated in the figure, where the assay was carried out using tubercidin or ribavirin as a control.

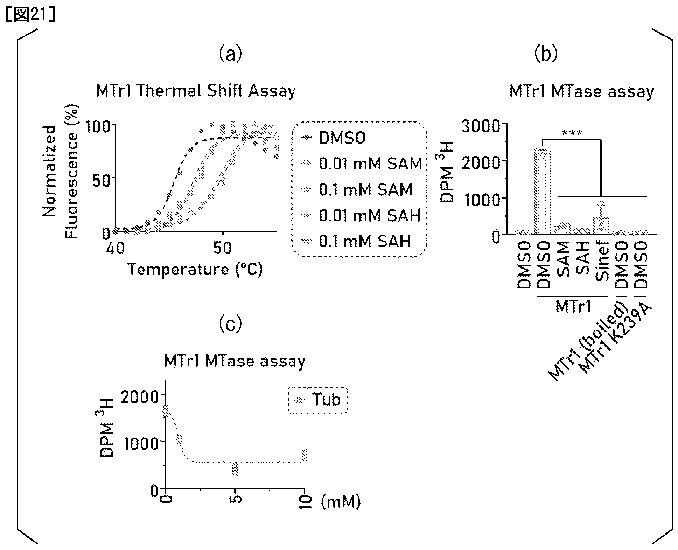

FIG. 21(a) shows a graph showing other results of the thermal shift assay of the recombinant MTr1 using SYPRO Orange. The assay was carried out at the concentration indicated in the figure, where the assay was carried out using SAM or SAH.

From these results, it was confirmed by the thermal shift assay that tubercidin directly binds to the recombinant MTr1.

FIG. 21(b) shows a graph showing the results of an MTase assay using human MTr1 WT or MTr1 K239A, which contains 0.1 mM of SAM, SAH, or sinefungin.

FIG. 21(c) shows a graph showing the results of the MTase assay using human MTr1 containing tubercidin at the indicated concentration.

The statistics were calculated using a two-sided Student's t-test (***p < 0.001).

These results indicate that, importantly, tubercidin inhibits the MTase activity of the recombinant MTr1 protein in a dosage-dependent manner, and tubercidin is an inhibitor of MTr1 that suppresses IAV replication.

It is known in the related art that tubercidin has a plurality of activities including antiviral activity and anticancer activity; however, in addition, it is also known that tubercidin exhibits a certain toxicity in vivo.

Accordingly, in order to identify a more excellent tubercidin-related compound, we evaluated 115 tubercidin-related compounds by an antiviral drug assay according to a three-stage screening procedure. A search was carried out for compounds having a specific anti-IAV and anti-IBV activity and having minimal or no toxicity in vitro and in vivo.

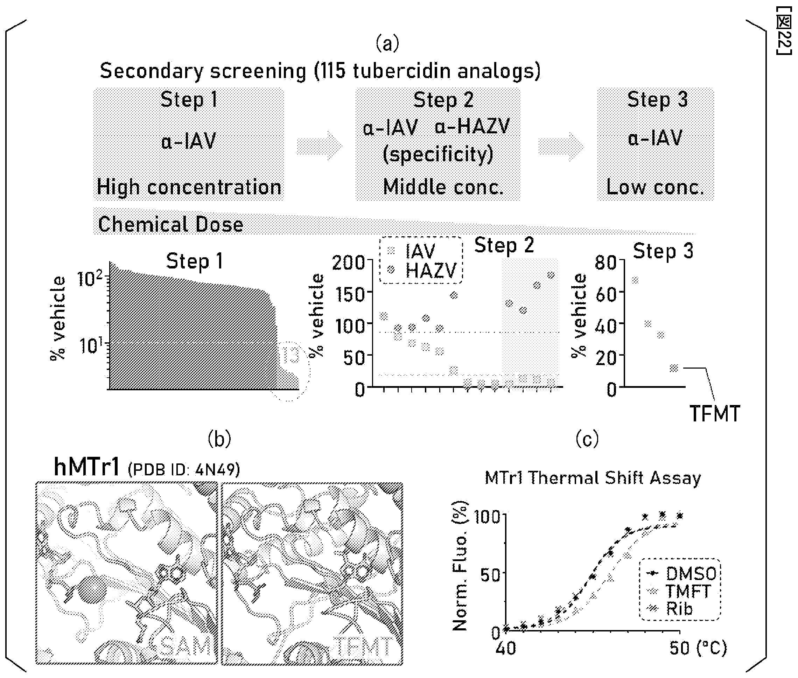

FIG. 22(a) shows a schematic flowchart of a secondary screening process consisting of three steps.

Each compound was added at concentrations of 100 µM (step 1), 10 µM (step 2), and 1 µM (step 3) as the quantification of the GFP level in A549 cells infected with IAV-GFP (m.o.i., 0.1) for 19 hours. qRT-PCR analysis of HAZV RNA in A549 cells infected with HAZV (m.o.i., 0.1) was also carried out in the step 2.

In the step 1, the cells were infected with IAV in the presence of the individual tubercidin-related compounds, and 13 compounds having an anti-IAV effect were identified. In the step 2, in order to exclude the viral infection or the off-target inhibition of toxicity, the anti-IAV activity at a lower concentration was investigated, and counter screening was carried out for the activity against HAZV.

Interestingly, similarly to the results in the MTr1 KO cells described later, four compounds that inhibit IAV replication without reducing HAZV replication in the step 2 were found.

In the step 3, it was evaluated which of the specified compounds could inhibit at the lowest concentration, and finally, trifluoromethyl tubercidin (TFMT) was identified as the most effective compound.

Next, the binding of TFMT to the SAM binding pocket of each of MTr1 and recombinant MTr1 was checked by an in silico docking and an in vitro thermal shift assay.

FIG. 22(b) shows a schematic view showing a docking state of TFMT in a SAM binding pocket of human MTr1.

FIG. 22(c) shows a graph showing the results of a thermal shift assay of Mtr1 with 1 mM TFMT or ribavirin, which uses SYPRO Orange.

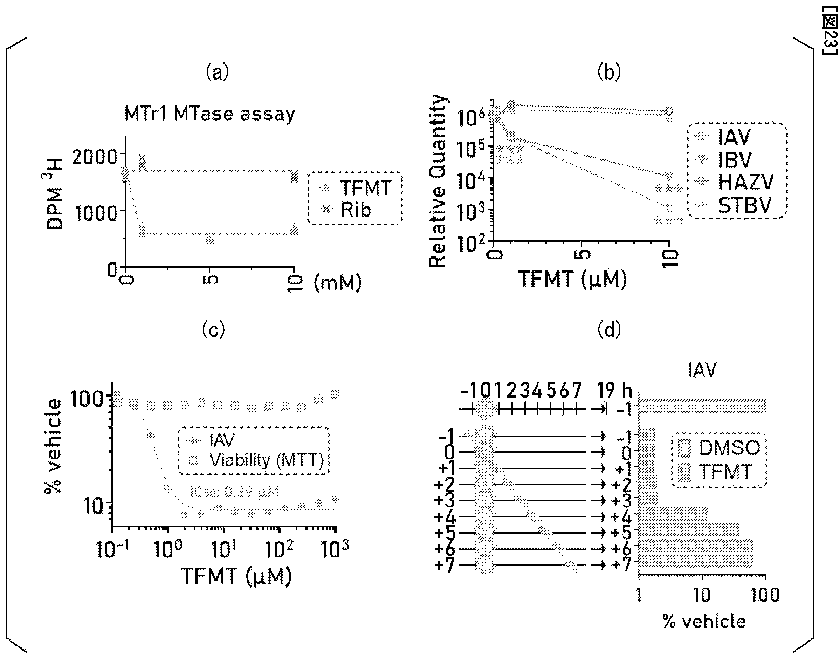

FIG. 23(a) shows a graph showing the results of an MTase assay of MTr1 with TFMT or ribavirin having each of the concentrations indicated in the figure.

From the results in the figure, it was confirmed that the MTase activity of the recombinant MTr1 protein is inhibited by TFMT.

The specificity of the antiviral activity was also investigated by using two additional viruses, IBV and STBV.

FIG. 23(b) shows a graph showing the results of the qRT-PCR analysis of each virus RNA. It shows qRT-PCR analysis of the virus RNA indicated in the figure in A549 cells, which have been infected with IAV (m.o.i., 1), IBV (m.o.i., 1), HAZV (m.o.i., 0.1), or STBV (m.o.i., 0.1) for 24 hours.

Importantly, the obtained results were such that TFMT exhibits an antiviral activity against IAV and IBV but does not exhibit an antiviral activity against HAZV and STBV. This is a point that exactly matches the above-described phenotype of the Mtr1 deficiency.

FIG. 23(c) shows a graph showing the results of quantification of the GFP level in A549 cells infected with IAV-GFP. It shows the IAV replication level of A549 cells infected with IAV-GFP (m.o.i., 0.1) for 19 hours and the quantification of the GFP level in the cell cytotoxicity test (WST-8).

In a case of being measured in the WST-8 cell survival rate assay, the IC50 of TFMT for IAV infection was 0.30 µM, and no significant in vitro toxicity was confirmed within the effective concentration range.

FIG. 23(d) shows a graph showing the results of another quantification of the GFP level in A549 cells infected with IAV-GFP. The quantification of the GFP level in A549 cells infected with IAV-GFP (m.o.i., 0.1) for 19 hours is shown, and TFMT was added at 10 µM at a designated moment before or after infection. In B, D, I, and J, each of the compounds indicated in the figure was added 3 hours before the infection. The statistics were calculated using a two-sided Student's t-test (***p < 0.001).

It is worthy of notice that a TFMT treatment significantly inhibited IAV replication even in a case where TFMT was administered 3 to 4 hours after infection. However, in a case where the drug was administered later, the effect was reduced or could not be confirmed.

### [Test Example 3: Verification of efficacy in normal human bronchial epithelial cells, human lung explant, and mouse]

Next, the inventors of the present invention examined the anti-IAV activity of TFMT in normal human bronchial epithelial (NHBE) cells.

FIG. 24(a) shows a graph showing the results of qRT-PCR analysis of IAV RNA (segment 7) in normal human bronchial epithelial (NHBE) cells infected with the IAV PR8 strain at an m.o.i. of 1 for 24 hours. In FIG. 24(a) to FIG. 24(d), TFMT was added 3 hours before the infection.

FIG. 24(b) shows a photographic view showing the results of Western blot analysis of indicated proteins in NHBE cells infected with the IAV PR8 strain at an m.o.i. of 1 for 24 hours.

As shown in the figure, the RNA level and the protein level of IAV (H1N1 or PR8) were significantly reduced in a dosage-dependent manner by the TFMT treatment.

FIG. 24(c) shows a photographic view showing the results of immunostaining of NHBE cells infected with the IAV PR8 strain. Immunostaining of IAV NP was carried out 24 hours (m.o.i. 2) after infection in the presence of 10 µM TFMT. The bar in the figure has a length of 100 µm.

It was also revealed by histological analysis that the IAV NP level in the TFMT-treated NHBE cells is significantly decreased without cell cytotoxicity.

FIG. 24(d) shows a graph showing the results of qRT-PCR analysis of HAZV RNA in NHBE cells infected with HAZV. NHBE cells infected with HAZV for 24 hours at an m.o.i. of 0.1 were analyzed.

Importantly, the TFMT treatment did not inhibit HAZV replication. This indicates that the specific efficacy of this compound against a specific virus is also maintained in human primary cells.

Since this compound was effective in human NHBE cells, the evaluation of TFMT in a human lung explant was carried out ex vivo.

FIG. 24(e) shows a schematic view of IAV infection in a human lung explant. The lung tissue was infected with IAV (H1N1, seasonal isolated strain in 2019), and the virus titer in the supernatant at the indicated moment was measured by a plaque assay.

FIG. 24(f) shows a graph showing the results of the measurement of the virus proliferation and the virus titer of seasonal IAV in the human lung tissue in one donor.

FIG. 24(g) shows a graph showing the results of the measurement of the virus proliferation and the virus titer of seasonal IAV in the human lung tissue in 6 independent donors.

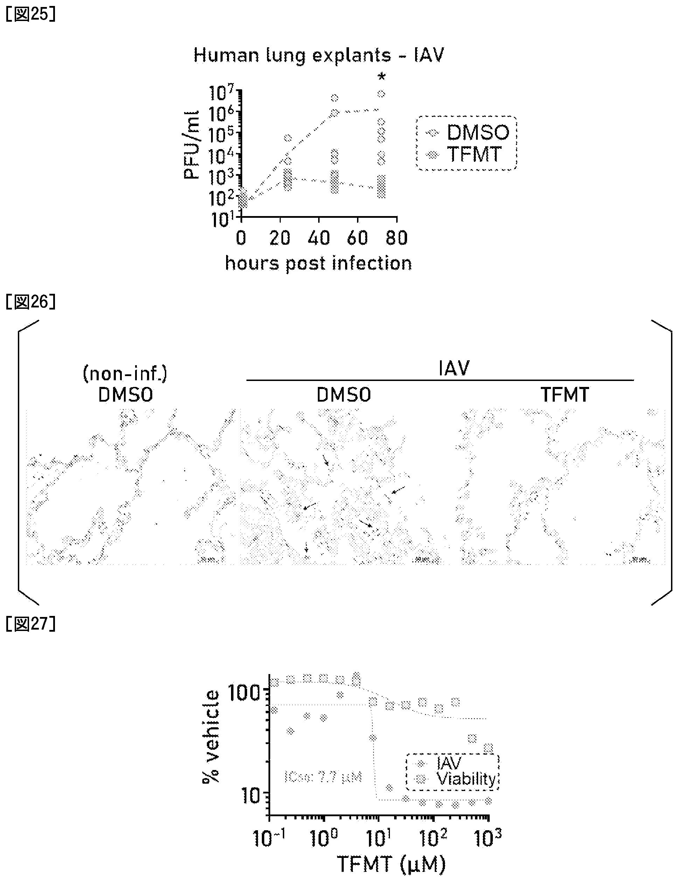

FIG. 25 shows a graph showing the virus proliferation of seasonal IAV (H1N1 from 2019) in the human lung tissue (n = 6). After the infection, the supernatant was collected at the indicated moment, and the virus titer was measured by a plaque assay. TFMT was added at 10 µM before and after infection.

The titer of the untreated sample increased to more than 105 PFU/ml at 48 or 72 hours after infection; however, the titer from TFMT-treated lung explant remained less than 103 PFU/ml and was 100 to 1,000. In addition, the titers of the independent donors of all six persons were summed up, which showed a significant difference between the titer in the culture supernatant as a control and the IAV titer reduced by the TFMT treatment.

FIG. 26 shows a photographic view showing immunohistochemistry of virus proliferation and the seasonal IAV (H1N1 from 2019) protein in the human lung tissue. The tissues were stained using an anti-NP antibody. TFMT was added at 10 µM before and after infection.

In the IAV-infected lung tissue which was treated with TFMT and infected with IAV, the IAV NP-positive cells and morphological changes were not observed, which was consistent with the virus titer. This indicates that TFMT has a high ability to disable the replication of the seasonal IAV isolated strain ex vivo and indicates the possibility of being applied in clinical treatment.

In order to examine the effectiveness of this compound in vivo, further verification was carried out using a mouse system.

FIG. 27 shows a graph showing the results of a cell cytotoxicity test and the level of GFP in LA-4 cells infected with IAV-GFP. The infection was carried out with IAV-GFP (m.o.i., 1) for 19 hours, and the quantification of the GFP level and the cell cytotoxicity test (WST-8) were carried out. TFMT was added at the concentration indicated in the figure 3 hours before the infection. The statistics were calculated using a Wilcoxon matched-pairs signed-rank test for the human lung explant assay (*p < 0.05).

From the figure, it was confirmed that the IAV-infected mouse cell line LA-4 also maintains the inhibitory activity even although TFMT has a lower efficacy than that of the human cell (IC50: 7.7 µM).

Next, the in vivo toxicity in the mouse was evaluated by carrying out nasal cavity inoculation once a day for two days.

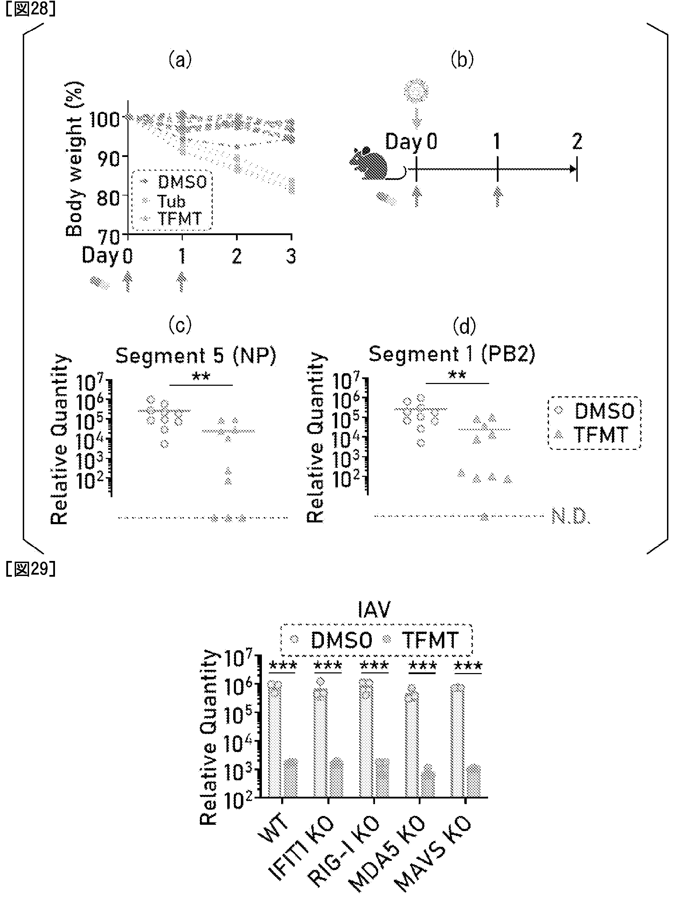

FIG. 28(a) shows a graph showing a change in body weight in a case where nasal cavity inoculation has been carried out on a mouse. TFMT or tubercidin at 2 mg/kg was intranasally administered to C57BL/6 mice on the 0th day of infection and the 1st day of infection (indicated by arrows in the figure).

Tubercidin is known to have a certain toxicity, and treatment with tubercidin caused a significant body weight loss in mice. On the other hand, the obtained results were such that the selected derivative TFMT does not cause body weight loss or cell cytotoxicity.

Further, the effect of the TFM treatment under various conditions was examined.

FIG. 28(b) shows a schematic view of an IAV infection model in an in vivo system of a mouse. As shown in the figure, the IAV WSN and TFMT were introduced into the nasal cavity. TFMT was added on the 0th day of the IAV WSN infection and the 1st day of the IAV WSN infection.

FIG. 28(c) shows a graph showing the results of qRT-PCR analysis of the segment 5 of IAV RNA in the lung tissue.

FIG. 28(d) shows a graph showing the results of qRT-PCR analysis of the segment 1 of IAV RNA in the lung tissue.

qRT-PCR analysis (5 × 10³ PFU) of IAV RNA in the lung tissue of the IAV-infected C57BL/6 mice on the 2nd day was carried out. TFMT of 2 mg/kg was intranasally administered on the 0th day of infection and the 1st day of infection. The statistic was calculated using a two-sided Student's t-test for the assay of NHBE cells, a Mann-Whitney test for the in vivo assay in mice, and a Wilcoxon matched-pairs signed-rank test for the ex vivo assay of the lung in humans. (*p < 0.05, **p < 0.01).

At 2 days after infection by the indicated TFMT treatment, the IAV NP level and the PB2 mRNA level in the mouse lung were significantly reduced by the TFMT treatment. This indicates that the anti-IAV effect is maintained although the in vivo toxicity is eliminated by the trifluoromethyl substitution of tubercidin.

Overall, the TFMT treatment showed the possibility of inhibiting IAV replication in all tested systems including human cell lines and NHBE cells in vitro, human lung explants ex vivo, and mice in vivo.

### [Test Example 4: Inhibition of interaction between host cap RNA and polymerase of virus by synergistic effect of antiviral activity of TFMT and approved anti-influenza drug]

Next, the mechanism of action in which TFMT treatment specifically inhibits the replication of IAV and IBV but does not inhibit the replication of other viruses was further verified in detail.

The following tests showed that TFMT inhibits IAV replication independently of the innate immune response, as well as the interaction between the IAV polymerase subunit PB2 and the capped RNA, and the synergistic effect with BXM. In addition, it was shown that MTr1 is essential for IAV replication in a method that does not depend on IFIT1 or RIG-I, and it is not necessary for the replication of SeV, VSV, and EMCV. In addition, the structure modeling of the influenza virus polymerase and the induction of mutation are shown.

FIG. 29 shows a graph showing the results of qRT-PCR analysis of IAV RNA in A549 cells infected with the IAV PR8 strain. The infection was carried out with the IAV PR8 strain at an m.o.i. of 1 for 24 hours, and IAV RNA (segment 7) was analyzed.

The effect of the TFMT treatment on IAV replication was independent of the IFIT1-dependent isolation of RNA or RIG-I/MDA5 signaling.

In addition, the IAV replication level in the RIG-I/MTr1 or IFIT1/MTr1 double KO cells was examined.

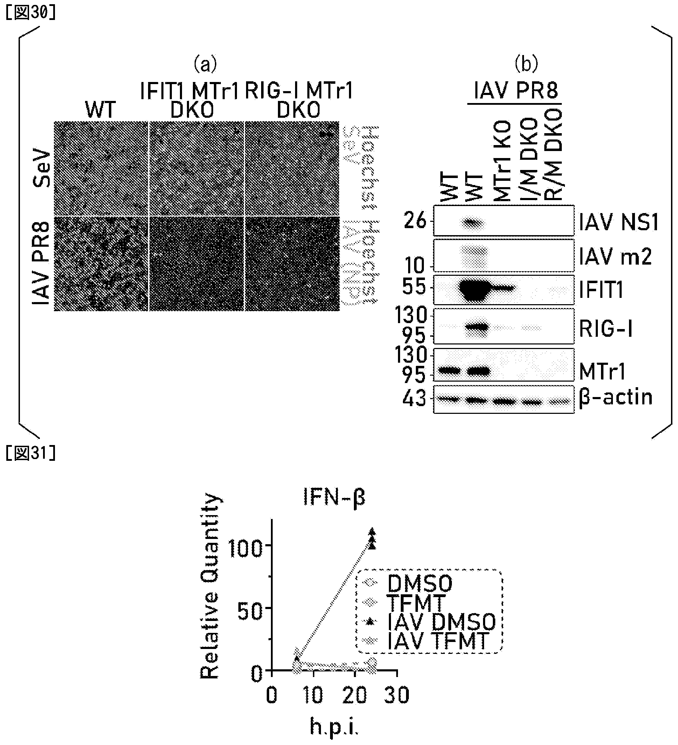

FIG. 30(a) shows a photographic view showing immunostaining of a viral protein of A549 cells infected with SeV or IAV. A549 cells infected with SeV (1 × 10⁻⁵ HAU/cell) or IAV PR8 (m.o.i., 10) for 20 hours were subjected to immunostaining of a viral protein in the genotype indicated in the figure.

FIG. 30(b) shows a photographic view showing the results of Western blot analysis of viral proteins of A549 cells infected with SeV or IAV. A549 cells infected with the IAV PR8 strain at an m.o.i. of 1 for 26 hours were subjected to the detection of the protein in the genotype indicated in the figure.

As shown in the figure, the IAV replication was not detected as in the case of the MTr1 KO cells, and this indicates that there is no involvement of the immune receptor RIG-I or the antiviral protein IFIT1 in the TFMT-dependent suppression of IAV replication by the blocking of MTr1.

FIG. 31 shows a graph showing the results of qRT-PCR analysis of IFN-β RNA in A549 cells. The IFN-β RNA in the IAV PR8 strain-infected A549 cells at the moment indicated in the figure at IAV: m.o.i. 1 was analyzed in the presence or absence of 10 µM TFMT.

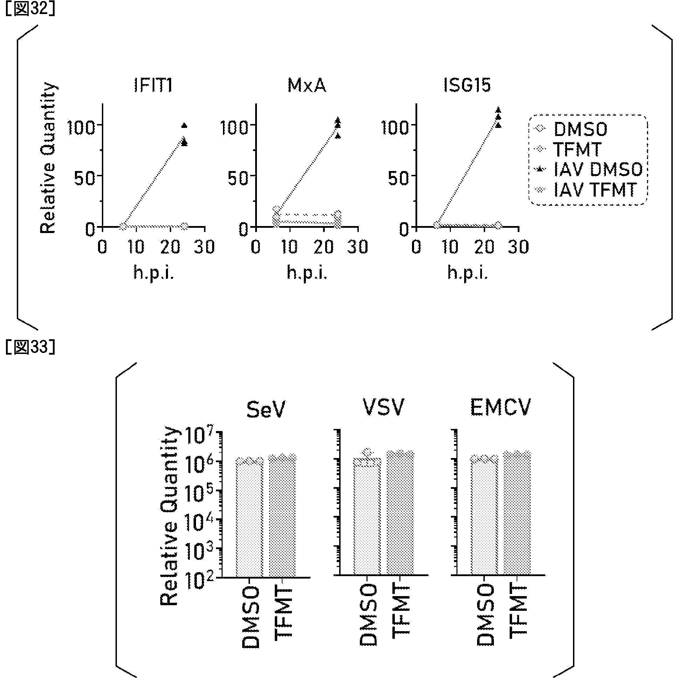

FIG. 32 shows a graph showing the results of qRT-PCR analysis of each of other RNAs in A549 cells. Each RNA indicated in the figure at the indicated moment at the m.o.i. of 1, in A549 cells which had been infected or not infected with the IAV PR8 strain in the presence or absence of 10 µM TFMT, was analyzed.

The treatment with TFMT did not induce IFN-β or antiviral ISG. This further emphasizes that the observed antiviral effect does not depend on the innate immune activation.

FIG. 33 shows a graph showing the results of qRT-PCR analysis of each RNA in A549 cells infected with other RNA viruses. SeV, VSV, or EMCV RNA (segment 7) in A549 cells after 24 hours from infection with SeV (1 × 10⁻⁶ HAU/cell), VSV-GFP (m.o.i., 1), or EMCV (m.o.i., 0.01) was analyzed.

The replication level of the IFN-sensitive non-cap-snatching RNA viruses such as Sendai virus (SeV), VSV, and EMCV was not changed by the treatment with TFMT, which is consistent with the above results.

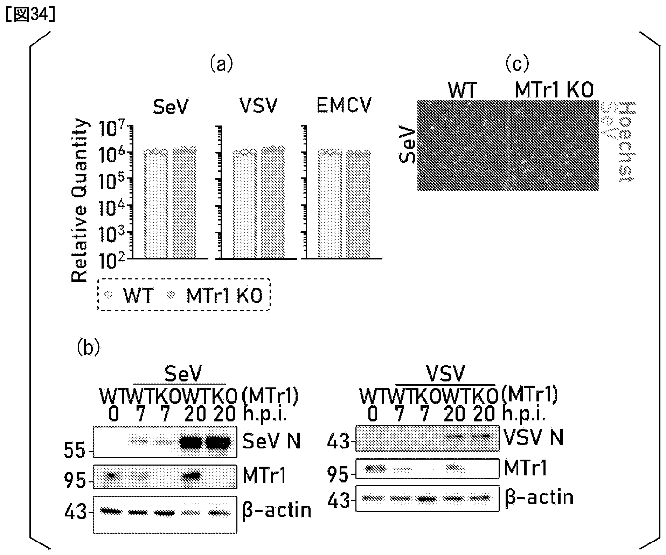

FIG. 34(a) shows a graph showing the results of qRT-PCR analysis of each virus RNA in MTr1 KO A549 cells infected with each virus. Each virus RNA in the figure in WT and MTr1 KO A549 cells, which had been infected with SeV (1 × 10⁻⁶ HAU/cell), VSV (m.o.i., 1), or EMCV (m.o.i., 0.1) for 26 hours, was analyzed. It is noted that in FIG. 34(a) to FIG. 34(c), TFMT was added at 10 µM 3 hours before the infection.

FIG. 34(b) shows a photographic view showing the results of Western blot analysis of each protein in MTr1 KO A549 cells infected with each virus. SARS-CoV-2 (m.o.i., 0.1) at the moment indicated in the figure after infection with SeV (1 × 10⁻⁶ HAU/cell) or VSV GFP (m.o.i., 1) and 24 hours after infection was analyzed.

FIG. 34(c) shows a photographic view showing the results of immunostaining of a viral protein in MTr1 KO A549 cells infected with SeV. The viral proteins in WT and MTr1 KO A549 cells, which had been infected with SeV (1 × 10⁻⁶ HAU/cell) for 12 hours, were analyzed.

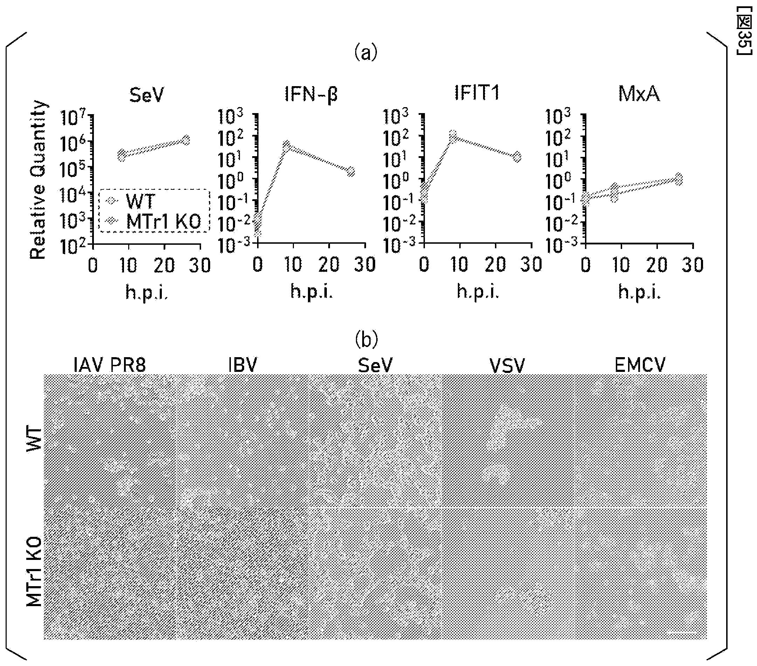

FIG. 35(a) shows a graph showing the results of qRT-PCR analysis of a virus and host RNA in MTr1 KO A549 cells infected with SeV. The virus and the host RNA in WT and MTr1 KO A549 cells, which have been infected with SeV (1 × 10⁻⁶ HAU/cell) at the moment indicated in the figure, were analyzed.

FIG. 35(b) shows a photographic view showing optical microscopic images of MTr1 KO HEK-293T cells infected with each virus. WT and MTr1 KO HEK-293 cells, which had been infected with IAV PR8 (m.o.i., 1), IBV (m.o.i., 1), VSV (m.o.i., 10), SeV (1 × 10⁻⁶ HAU/cell), and EMCV (m.o.i., 0.001), were observed on the 2nd day after infection. The bar in the figure has a length of 50 µm.

In a case where these viral infections in the MTr1 KO cells were examined, it was confirmed that there is no difference in the level of virus replication.

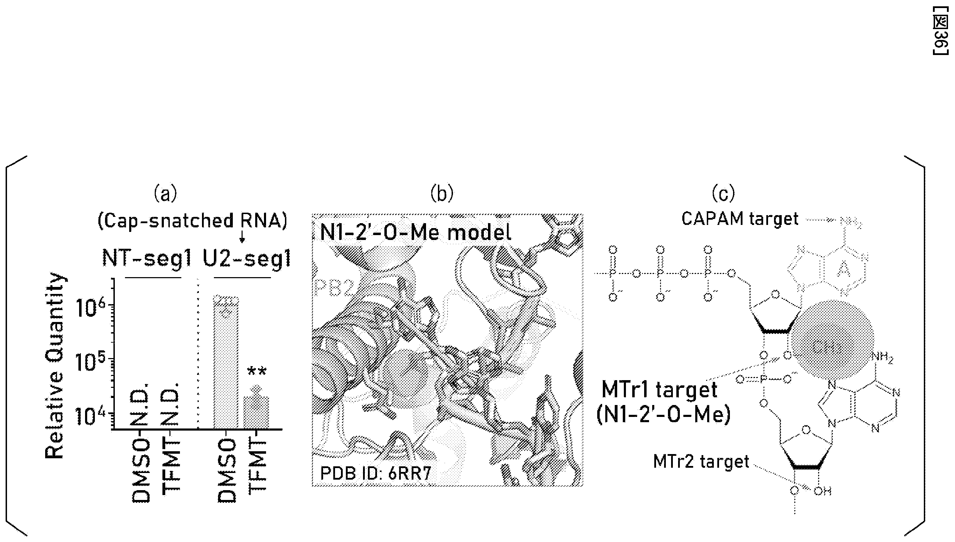

FIG. 36(a) shows a graph showing qRT-PCR analysis of NT/U2-IAV hybrid RNA in A549 cells infected with IAV PR8. The NT/U2-IAV hybrid RNA in A549 cells infected with IAV PR8 for 24 hours at an m.o.i. 1 was analyzed.

As in the case of the MTr1 deficiency, the TFMT treatment decreased the expression level of the IAV mRNA (segment 1), which had been specifically snatched from the U2 spliceosome snRNA. These results indicate that TFMT treatment inhibits IAV replication by directly affecting the cap snatching activity of IAV, rather than by immunomodulation.

In order to further clarify the mechanism by which the loss of N1-2'-O-Me of the host cap RNA in the TFMT treatment causes the IAV cap snatching defect, the interaction site of N1-2'-O-Me in the polymerase PB2 subunit of IAV was analyzed.

FIG. 36(b) shows a schematic view of an interaction site of N1-2'-O-Me. A structural model (PDB ID: 6RR7) of N1-2'-O-Me in RNA bound to IAV PB2 is shown. A portion surrounded by a circle indicated by an arrow indicates a methyl group at the N1-2'-O position.

FIG. 36(c) shows a structure of N1 and N2 of the cap1 RNA, where N1-2'-O-Me is shown in the same manner.

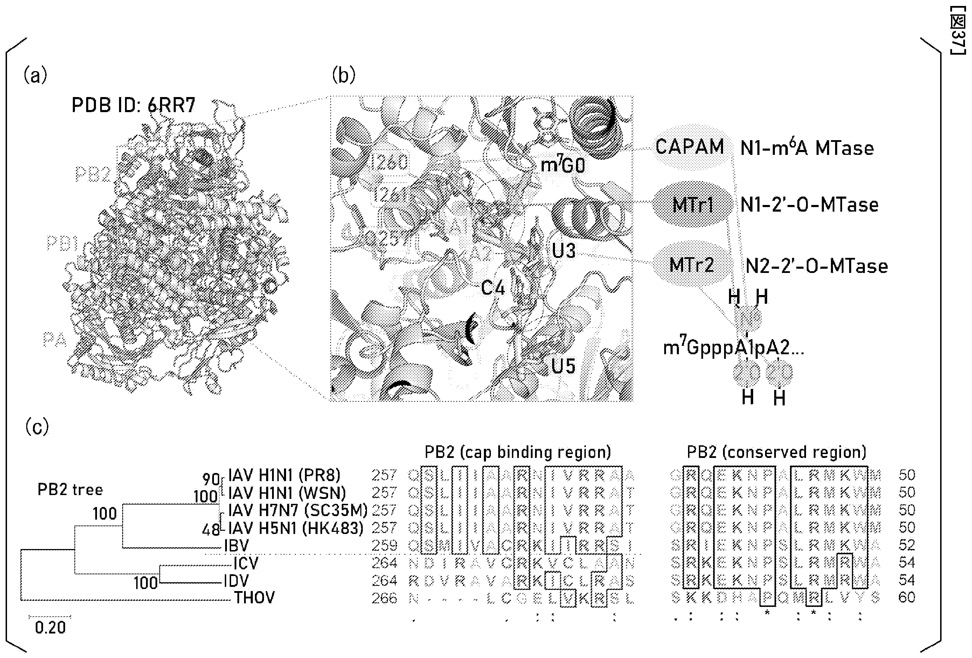

FIG. 37(a) shows a schematic view of a structure of an IAV polymerase that forms a complex with RNA. IAV polymerase (PDB ID: 6RR7)

FIG. 37(b) shows a schematic view showing the RNA binding region of PB2 in an enlarged manner. The target site of MTr1, MTr2, or CAPAM is shown in the figure.

FIG. 37(c) shows a schematic view showing a phylogenetic tree of PB2 in influenza virus and THOV and partial sequence alignment of amino acid residues (RNA cap-binding region and conserved region).

The above figure shows a calculation model of virtual addition of N1-2'-O-Me, which has been constructed based on the reported structure of the IAV polymerase complex with the cap0 RNA (PDB ID: 6RR7). As a result, it was revealed that N1-2'-O-Me is located close (within 3 Å) to Q257 and I261, which are two amino acids of the α-helix structure of PB2. As a result, it is presumed that the methylation increases the affinity between the helix of PB2 and the host mRNA through a hydrophobic effect and a van der Waals interaction between hydrophobic amino acids I260 and I261.

Therefore, it is considered that in a case where the N1-2'-O-methyl group of the capped RNA is not present in the MTr1 KO cells, the appropriate interaction with the PB2 subunit of the IAV polymerase is hindered. In addition to N1-2'-O-Me, two other well-known methylations, N2-2'-O-Me and N1-m6A, occur in the mature cap structure. Each of these methylation reactions is catalyzed by methyltransferase MTr2 and cap-specific adenosine methyltransferase (CAPAM, PCIF1).

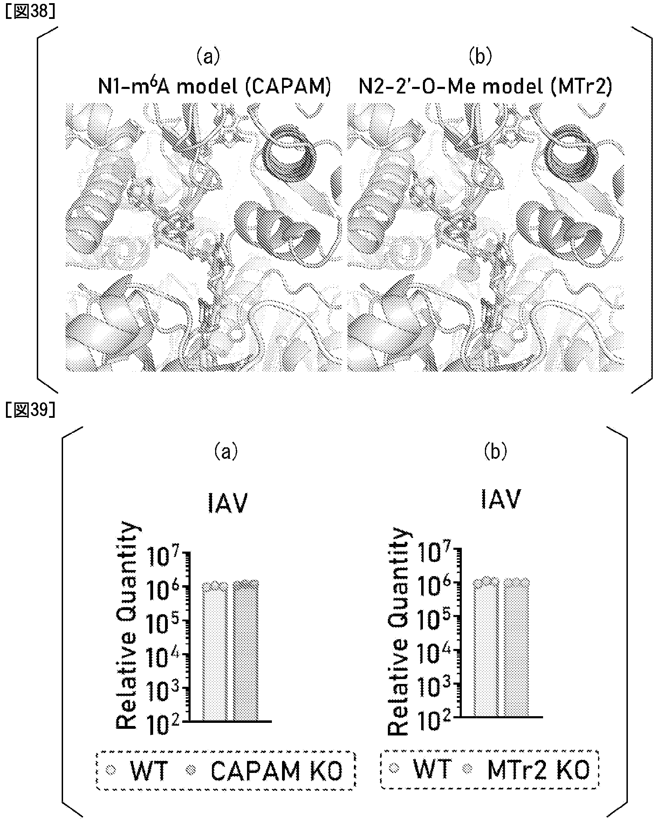

FIG. 38(a) shows a schematic view showing a structural model of N1-m6A in RNA bound to IAV PB2.

FIG. 38(b) shows a schematic view showing a structural model of N2-2'-O-Me in RNA bound to IAV PB2.

The possibility of steric collision of N1-m6A and N2-2'-O-methylation was simulated. In the figure, a site surrounded by a circular shape, which is indicated by an arrow, indicates an additional methyl group.

In particular, a significant interaction between PB2 and N2-2'-O-Me or N1-m6A was not observed.

FIG. 39 shows graphs showing qRT-PCR analysis of IAV RNA of A549 cells infected with IAV PR8. FIG. 39(a) shows analysis for CAPAM KO cells, and FIG. 39(b) shows analysis for Mtr2 KO cells. The IAV RNA (segment 7) in the indicated genotype of A549 cells infected with IAV PR8 for 24 hours at an m.o.i. 1 was analyzed.

The IAV replication defect was not observed in CAPAM KO cells or MTr2 KO cells, which is consistent with the above data.

In order to experimentally show that the depletion of MTr1 affects the interaction between PB2 and the cap RNA, immunoprecipitation of mRNA was carried out through a poly(A) binding protein (PABP).

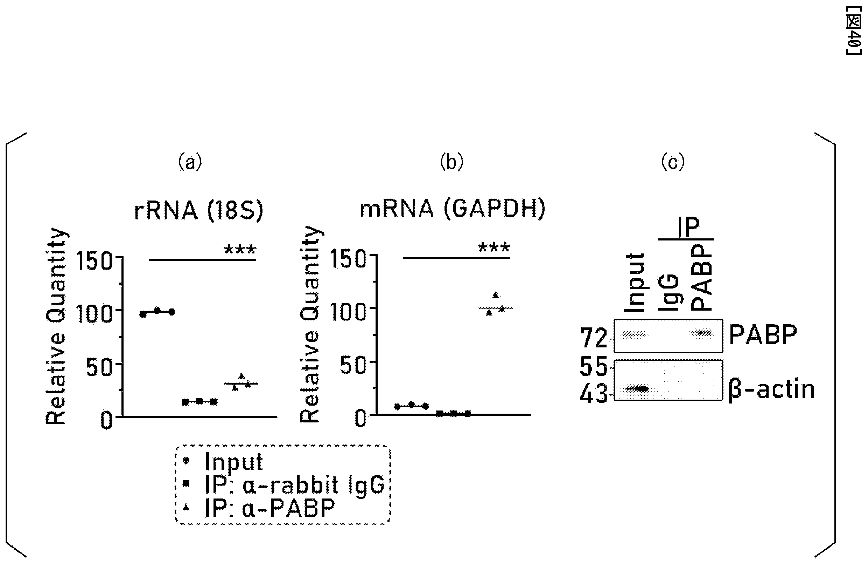

FIG. 40 shows graphs showing the results of qRT-PCR analysis of ribosomal RNA (18S) and mRNA (GAPDH) in the input and the immunoprecipitated RNA from HEK-293T cells. FIG. 40(a) shows ribosomal RNA (18S) and FIG. 40(b) shows mRNA (GAPDH). The amount of each RNA was 10 ng.

FIG. 40(c) shows a photographic view showing the results of the Western blot analysis of the PABP protein that has been subjected to the PABP immunoprecipitation.

First, it could be confirmed by using RT-PCR that mRNA is concentrated in the immunoprecipitated fraction of PABP.

Next, PABP immunoprecipitation was carried out using the IAV PB2 overexpressing control cell or the MTr1 KO cell.

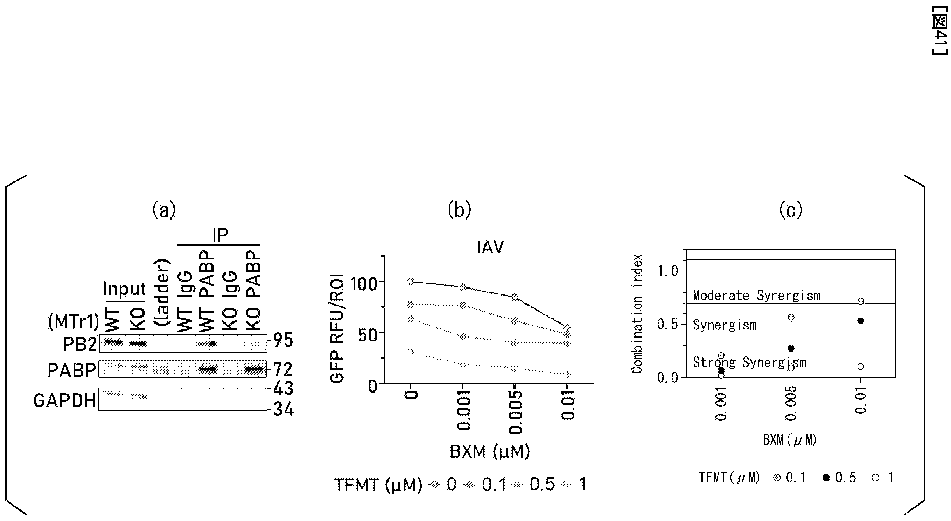

FIG. 41(a) shows a photographic view showing the results of Western blot analysis of input and immunoprecipitated proteins in PB2-expressing WT cells and MTr1 KO HEK-293T cells.

It shows that in the MTr1-deficient cells, the amount of PB2 which has coprecipitated in association with PABP immunoprecipitation is significantly reduced, and the presence of MTr1-dependent N1-2'-O-Me significantly increases the affinity of PB2 for the cap. This result is consistent with the result of mRNA in the cell and the result of the structure modeling.

Baloxavir marboxil (BXM) is an approved anti-IAV drug, which targets the active site of the polymerase PA subunits 18 and 19. TFMT affects the cap-binding activity of different subunits of the IAV polymerase PB2. Therefore, a potential synergistic effect on the anti-IAV activity was examined.

FIG. 41(b) shows a graph showing a synergy effect of BXM and TFMT. It shows the relative fluorescence units of GFP in A549 cells infected with IAV SC35M-GFP at 0.1 infectious particles/cell for 19 hours. TFMT and/or BXM was added at a designated concentration 3 hours before the infection.

FIG. 41(c) shows the results of calculating the combination index as a scale of the synergy evaluation.

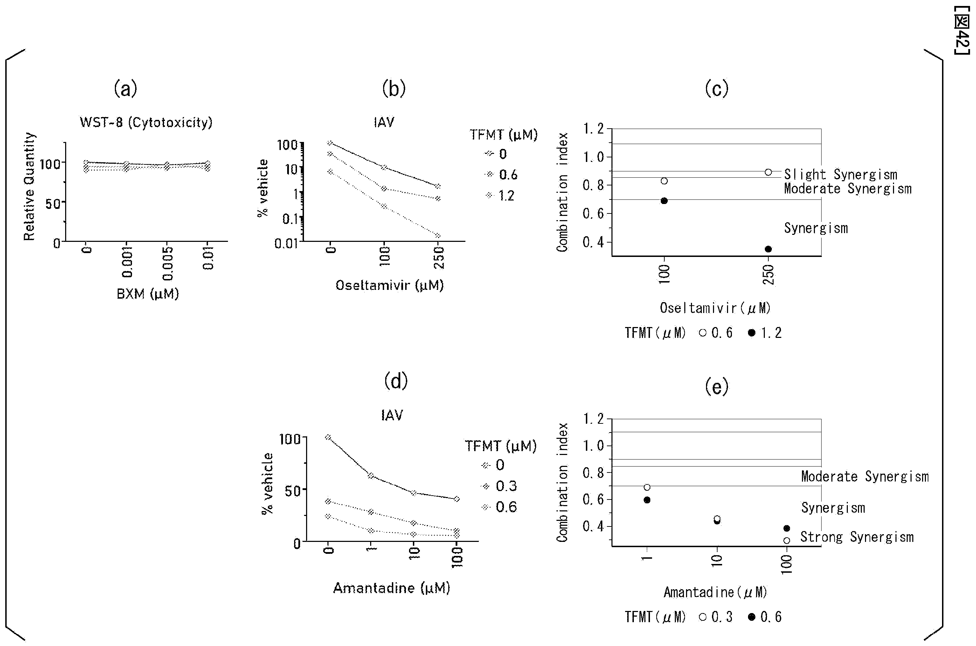

FIG. 42(a) shows a graph showing a WST-8 assay for FIG. 41.

Interestingly, in the combined treatment of TFMT and BXM, a strong synergistic effect was observed without cell cytotoxicity.

Further, a combination of TFMT, oseltamivir which is a neuraminidase inhibitor, and amantadine which is an m2 inhibitor also exhibited a synergistic effect.

FIG. 42(b) shows a graph showing a synergy effect of oseltamivir and TFMT. It shows qRT-PCR analysis of IAV RNA (segment 7) in A549 cells infected with the IAV PR8 strain at an m.o.i. of 0.01 for 55 hours. TFMT and/or oseltamivir was added at a designated concentration 1 hour before the infection.

FIG. 42(c) shows the results of calculating the combination index as a scale of the synergy evaluation in the previous figure.

FIG. 42(d) shows a graph showing a synergy effect of amantadine and TFMT. It shows the relative fluorescence units of GFP in A549 cells infected with IAV SC35M-GFP at 0.1 infectious particles/cell for 19 hours. TFMT and/or amantadine was added at a designated concentration 3 hours before the infection.

FIG. 42(e) shows the results of calculating the combination index as a scale of the synergy evaluation in the previous figure.

These data suggest the possibility of using TFMT in combination therapy.

The I38T mutant has been reported in the PA subunit of the BXM resistant IAV18. From this, it was examined whether this BXM resistant virus having an I38T mutation in the PA subunit could be replicated in the MTr1 KO cell.

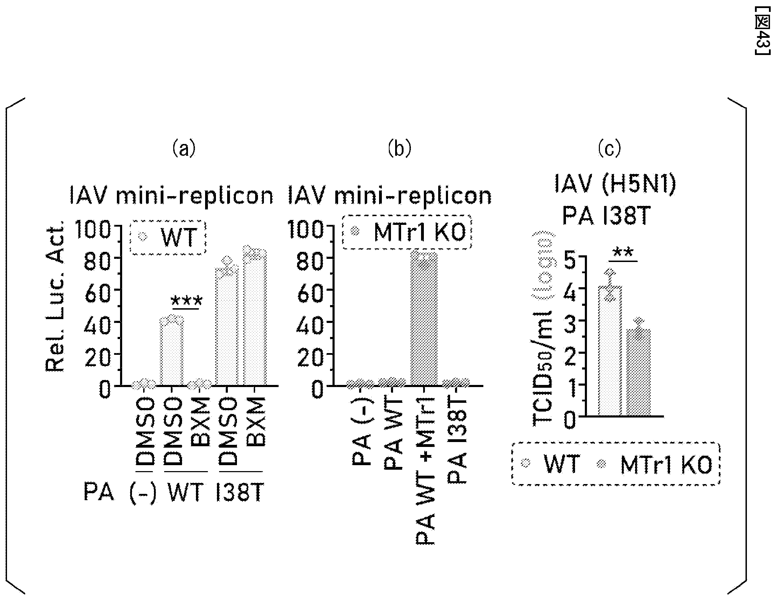

FIG. 43 shows graphs showing luciferase reporter assays of an IAV PR8 mini-replicon of Mtr1 KO HEK-293T which overexpresses each PA subunit. FIG. 43(a) shows the results of the wild type (WT), and FIG. 43(b) shows the results of the MTr1 KO cells. The results in WT and MTr1 KO HEK-293 cells, which overexpress IAV PB2, PB1, NP, and the indicated PA, are shown. 0.1 µM BXM was added 1 hour before transfection.

FIG. 43(c) shows a graph showing the virus quantity in the supernatant of MTr1 KO A549 cells infected with IAV HK483 PA I38T, which is a BXM resistant virus. WT and MTr1 KO A549 cells were infected with IAV HK483 PA I38T at an m.o.i. of 0.1 for 48 hours, and the quantity in the supernatant thereof is shown. The statistic was calculated using a two-sided Student's t-test (**p < 0.01, ***p < 0.001).

The IAV mini-replicon assay and the infection with the BXM-resistant H5N1 virus have revealed that the Mtr1 deficiency inhibits the replication of the BXM-resistant virus.

In consideration of the fact that the activity of the virus polymerase is extremely sensitive to the MTr1 deficiency, a question arises as to whether or not a specific IAV strain can overcome the strict requirement of N1-2'-O-Me at the cap-binding site of PB2.

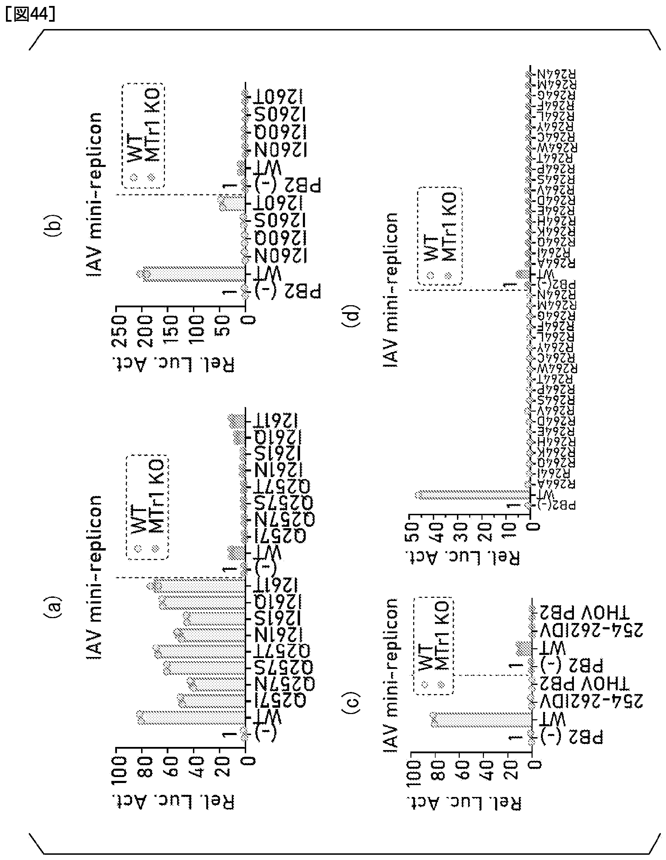

FIG. 44 shows graphs showing luciferase reporter assays of an IAV PR8 mini-replicon of MTr1 KO HEK-293T which overexpresses a PA subunit shown in the horizontal axis of each of FIG. 44(a) to FIG. 44(d). Cells that overexpress IAV PB1, PA, NP, and PB2 indicated in the figure are used.

It is noteworthy to mention that all the tested mutants having changes in the two interactive amino acid I260 and I261 did not support IAV replication in the MTr1 KO cells. Rather, the modeling approach according to the present embodiment suggests that the conserved amino acids are important for IAV replication in WT cells and suggests that the IAV PB2 subunit is unlikely to adapt to the absence of the N1-2'-O-Me cap RNA structure for efficient replication.

In summary of the above results, the above-described test results emphasize the possibility of TFMT as an Mtr1 inhibitor that specifically suppresses cap snatching. As a result, it has been shown that the replication of various IAV and IBV strains, including the seasonal H1N1 isolated strain and high pathogenic avian IAV resistant to BXM, is suppressed.

Mechanistically, TFMT causes MTr1 deficiency and accumulation of cap0 RNA, which impairs the binding of PB2 to the host cap RNA and reduces the effectiveness of the IAV polymerase that initiates cap snatching and RNA synthesis. TFMT targets polymerase subunits PB2 and PA, where drugs for the polymerase subunits PB2 and PA are different from each other. Therefore, TFMT exhibited a strong synergistic effect with BXM.

It is worthy of notice that this TFMT-dependent restriction of IAV is independent of the restriction by the innate immune response or RIG-I and IFIT1, and does not affect the replication of interferon-sensitive viruses such as VSV and EMCV. In addition, interestingly, it was revealed that in a case of comparing PB2 between influenza virus and THOV, the primary structure of the cap RNA binding region (N1-2'-O-Me interaction amino acid) of IAV PB2 is well conserved in IBV PB2 but is not well conserved in ICV, IDV, or THOV PB2.

Further, it has been reported that IAV requires 10 to 13 nucleotides for cap snatching, whereas THOV snatches an m7G cap residue at the 5' terminal. This different approach of the virus polymerase to cap snatching may be capable of explaining the specificity of the restriction of TFMT for IAV and IBV.

In addition, the above test results report all aspects of identifying TFMT as a novel, highly specific, and non-toxic Mtr1 inhibitor that specifically restricts the replication of the cap snatching-dependent viruses IAV and IBVV.

In contrast to tubercidin, an MTr1 inhibitor identified in the primary screening, the TFMT treatment is allowed in vitro and in vivo without toxicity or activation of a host immune response.

This compound is not only an inhibitor having a new mechanism of action but also belongs to a novel class of antiviral drugs having a specific effect on influenza A and B. Drugs currently approved for an IAV treatment against the viral proteins are available; however, mutant strain viruses resistant to these drugs have been reported against each existing drug. On the other hand, as demonstrated by an MEK inhibitor ATR-002 that directly affects virus replication and regulates inflammation, thereby having broad efficacy against RNA viruses such as influenza virus and SARS-CoV-2, the antiviral drug for a host has a low possibility of inducing drug resistance.

In consideration of the immune activation or toxicity, the effectiveness of the host-targeted MTr1 inhibitor TFMT makes it possible to provide a new therapeutic method for influenza A virus and influenza B virus.

While the preferred embodiments of the present invention have been described and illustrated as described above, it is to be understood that they are intended to be illustrative and should not be considered as limiting the present invention. Additions, omissions, substitutions, and other changes can be made without departing from the spirit or scope of the present invention. Accordingly, the present invention should not be considered as being limited by the descriptions described above and is limited only by the scope of the appended claims.

### INDUSTRIAL APPLICABILITY

According to the present invention, it is possible to obtain a novel anti-influenza virus agent that inhibits a mechanism of proliferation or infection of an influenza virus and thus has a target which is not a specific viral protein, where the novel anti-influenza virus agent does not cause the emergence of drug-resistant viruses. It is possible to obtain a medicine, a food, a beverage, a supplement, an agricultural chemical, a feed, and a cosmetic, which use the anti-influenza virus agent.

## Claims

1. An anti-influenza virus composition comprising, as an active ingredient:
tubercidin, a tubercidin derivative, or a salt thereof.

2. The composition according to Claim 1,
wherein the tubercidin derivative is a compound represented by General Formula (1), 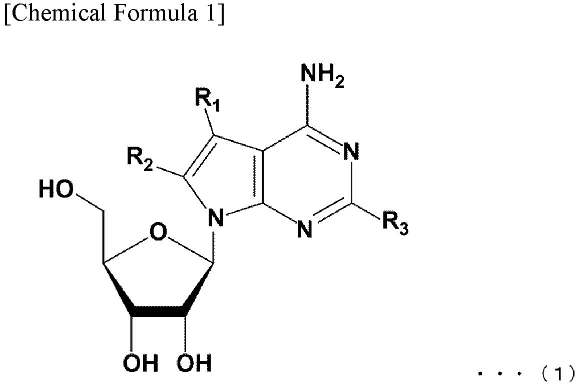 (in General Formula (1), R₁, R₂, and R₃ are each the same or different from each other and represent a hydrogen atom, a halogen atom, an alkyl group which may have a substituent, an alkenyl group which may have a substituent, an alkynyl group which may have a substituent, an alkoxy group which may have a substituent, an amino group which may have a substituent, an amide group which may have a substituent, a cyano group, a nitro group, a hydroxy group, a sulfone group, a cycloalkyl group which may have a substituent, an aryl group which may have a substituent, an aliphatic heterocyclic group which may have a substituent, or an aromatic heterocyclic group which may have a substituent).

3. The composition according to Claim 2,
wherein R₁ and R₂ are each the same or different from each other and represent a hydrogen atom, a halogen atom, an amino group which may have a substituent, an amide group which may have a substituent, or a cyano group, and
R₃ represents a hydrogen atom, an alkyl group which may have a substituent, an amino group which may have a substituent, or an aryl group which may have a substituent.

4. The composition according to Claim 2 or 3,
wherein R₃ represents a hydrogen atom.

5. The composition according to any one of Claims 2 to 4,
wherein R₁ and R₂ are each the same or different from each other and represent a halogen atom.

6. The composition according to any one of Claims 2 to 4,
wherein R₁ and R₂ are each the same or different from each other and represent an amino group which may have a substituent, or a cyano group.

7. The composition according to any one of Claims 2 to 4,
wherein R₁ represents an alkyl group substituted with a halogen.

8. The composition according to any one of Claims 2 to 4,
wherein R₁ represents a trifluoromethyl group.

9. The composition according to Claim 2,
wherein the tubercidin derivative is a compound represented by Formula (2),

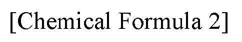

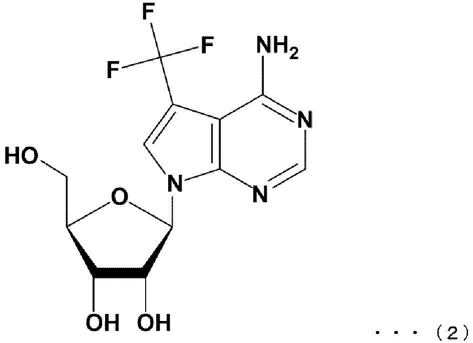

10. A medicine, a food, a beverage, a supplement, an agricultural chemical, a feed, or a cosmetic, comprising:
the composition according to any one of Claims 1 to 9.
